# EUROPEAN PATENT SPECIFICATION

(11) **EP 3 994 465 B1**
(45) Date of publication and mention of the grant of the patent: **04.09.2024**
(21) Application number: 20743037.2
(22) Date of filing: 01.07.2020
(51) Int. Cl.: G01N 33/53, G01N 35/00, G01N 35/04, G01N 35/10, G01N 35/02

(54) **CONFIGURABLE WASH PROCESS FOR A SAMPLE ANALYZER**
KONFIGURIERBARER WASCHVERFAHREN FÜR EINEN PROBENANALYSATOR
PROCÉDÉ DE LAVAGE CONFIGURABLE POUR UN ANALYSEUR D'ECHANTILLONS

(30) Priority: 03.07.2019 US 201962870673 P
(43) Date of publication of application: 11.05.2022
(73) Proprietor: Beckman Coulter, Inc., Brea, CA 92822 (US)
(72) Inventor: HOLMES, Laura Elizabeth Schilling, Chaska, MN 55318 (US); MIZUTANI, Takayuki, Chaska, MN 55318 (US); GUSTAFSON, Andrew John, Chaska, MN 55318 (US); TEAL, Donald, Chaska, MN 55318 (US); PRECORD, Cody Joseph, Chaska, MN 55318 (US)
(74) Representative: Müller-Boré & Partner Patentanwälte PartG mbB
(86) International application number: PCT/US2020/040480
(87) International publication number: WO 2021/003262

(56) References cited:
- EP-A2- 0 410 645
- WO-A1-2013/181019
- WO-A2-2005/008219
- WO-A2-99/57561
- US-A- 5 104 808
- US-A- 5 846 491
- US-A1- 2010 098 589

## Description

### CLAIM OF PRIORITY

This patent application claims the benefit of priority to U.S. Provisional Application Serial No. 62/870,673, filed July 3, 2019.

### BACKGROUND

This disclosure generally relates to the field of automatic substance preparation and evaluation instruments. In particular, the disclosure relates to methods and systems for evaluating a fluidic substance, e.g., a sample with/of bodily fluid, in a container. Such instruments typically process multiple samples sequentially in containers that may be disposable or non-disposable. The instrument may include one or more probes that distribute the fluidic substance, wash buffers, buffer solutions, reagents, and/or a variety of other fluids.

Automated clinical analyzers are well known in the art and are generally used for the automated or semi-automated analysis of patient samples. Typically, prepared patient samples, such as blood, are placed onto such an analyzer in sample containers, such as test tubes. The analyzer pipettes a patient sample and one or more reagents into a reaction cell (e.g., a reaction vessel or cuvette) where an analysis of the sample is conducted, usually for a particular analyte of interest, and results of the analysis are reported. Instruments known as UniCel^{®} DxI 600 Access^{®} Immunoassay System (i.e., DxI 600) and UniCel^{®} DxI 800 Access^{®} Immunoassay System (i.e., DxI 800), manufactured by Beckman Coulter, Inc. of Brea, California, USA, are examples of such automated clinical analyzers.

Automated pipettors are employed on such analyzers to transfer the patient samples, reagents, washing solutions, wash buffers, buffer solutions, substrates, and/or mixtures thereof as required for the analysis. Such pipettors can include a hollow probe having an open end or tip. The hollow probe is, for example, lowered into a sample container that holds a sample, a predetermined volume of the sample is withdrawn from the sample container, and the hollow probe is withdrawn from the sample container. The probe is moved, for example, to a position above a reaction cell, is again lowered, and the sample held in the hollow probe is expelled into the reaction cell. Similar actions may be used to pipette and deliver one or more reagents from reagent containers to the reaction cell, either with the same probe or with one or more reagent delivery probes. Similar actions may be used to pipette and deliver one or more washing solutions (i.e., wash buffers, buffer solutions, etc.) to the reaction cell and thereby clean (i.e., rinse) the reaction cell at various stages of the analysis. Similar actions may also be used to pipette and deliver one or more substrates to the reaction cell.

EP 0 410 645 A2 describes an automated analytical apparatus by which homogeneous and heterogeneous analyses can be performed concurrently and on a random access basis includes a circular reaction tray supporting a multiplicity of peripherally arranged reaction cuvettes, sample handling arrangement for supporting samples to be analyzed and a reagent tray supporting a plurality of liquid-reagent sources. The individual samples and liquid reagents are transferred, on a selective-random mode, by appropriate aspirating-dispensing apparatus into reaction cuvettes on the reaction tray which are successively positioned at sample and reagent addition stations.

US 2010/098589 A1 describes an error specifying method of specifying an error of an analyzer. The method specifies an error related to removal of a high-concentration reagent.

US 5 846 491 A describes an automated chemical analyzer capable of automatically analyzing a plurality of samples for at least two different analytes. The analyzer includes a plurality of assay resource stations and an analyzer control means. Each assay resource station includes an assay resource capable of performing a predetermined operation upon a sample-containing reaction vessel within a fixed indexing time.

WO 2005/008219 A2 describes a system and method for the simultaneous detection of multiple analytes in a sample. The detection system includes a housing that holds a reagent carousel rotatably coupled thereto. Further included in the housing is an incubator carousel rotatably coupled thereto. The housing also includes magnetic material that is associated with the incubation carousel for assisting in separation beads from reagent and wash solution.

WO 99/57561 A2 describes an automated analyzer for performing multiple diagnostic assays simultaneously including multiple stations, or modules, in which discrete aspects of the assay are performed on fluid samples contained in reaction receptacles. The analyzer includes stations for automatically preparing a specimen sample, incubating the sample at prescribed temperatures for prescribed periods, performing an analyte isolation procedure, and ascertaining the presence of a target analyte.

US 5 104 808 A describes a diagnostic instrument and method that indexes plural, sequentially actuated reaction vessels, stepwise to several processing positions. Several positions effecting wash have wash probes that are gauged together. Sample and/or reagent are not added to a number of vessels leading and trailing sample containing vessels.

WO 2013/181019 A1 describes methods and an apparatus adapted to wash magnetic particles isolated in a vessel. The methods include providing a vessel with wash liquid and a layer of magnetic particles, providing a probe having aspiration capability, positioning a probe tip above the layer of magnetic particles, aspirating at least some of the wash liquid above the layer, positioning the probe tip below the layer, and aspirating at least some of the wash liquid from below the layer of magnetic particles.

A variety of washing processes have been developed for use on such instruments to remove residues in the containers (e.g., samples, reagents, etc.). By washing the reaction cell, unattached portions of the sample, reagent, and/or substrate can be removed from the reaction cell. A common problem with reaction cell washing is residual unattached portions remaining in the reaction cell despite washing. This residue can interfere with subsequent analyses and thereby can falsely generate signals and provide incorrect results.

Another problem with reaction cell washing is unintentional washing of attached portions of the sample, reagent, and/or substrate from the reaction cell.

Thus, there is a need for a reaction cell washing arrangement and method of use of such an arrangement that overcomes these limitations of the prior art reaction cell washing approaches. There is further a need for a process for washing a reaction cell that overcomes the limitations of the prior art reaction cell washing processes. The needed improvements include, but are not limited to, removing residue from the reaction cell without removing portions of the sample, reagent, and/or substrate from the reaction cell that are desired to be kept in the reaction cell for analysis. The underlying technical problem is solved by the subject-matter according to independent claim 1. Additional embodiments are defined in the dependent claims.

### SUMMARY

The invention relates to an immunoassay diagnostic system configured to perform a plurality of assay types and thereby detect analytes in patient samples by at least combining each of the patient samples with at least one reagent and then washing away at least unreacted components of the patient sample according to claim 1. According to certain aspects of the present disclosure, a reaction cell washing arrangement includes a hollow probe configured to wash away at least some unreacted components of a patient sample from a reaction cell by a multiple number of wash actions. The washing arrangement is configured to wash the reaction cell within a predetermined timed sequence. The washing arrangement is configured to set the number of the wash actions to correspond with an assay type of the assay being performed within the reaction cell. Even though the number of wash actions may vary from test to test (i.e., assay to assay), the test throughput remains the same, and the overall test process speed is uncompromised.

According to certain aspects of the present disclosure, a sample analysis system includes at least a reaction cell washing arrangement for cleaning the reaction cell.

According to certain other aspects of the present disclosure, the sample analysis system may further include a cleaning fluid supply for supplying cleaning fluid.

According to certain additional aspects of the present disclosure, a carrier of the sample analysis system may include a rotating ring and/or a rotating disk with a plurality of holders for individually transporting a plurality of the reaction cells. A probe path of the hollow probe may be a linear path. The probe path may be a vertical path. In certain embodiments, the probe may move only along the probe path when the sample analysis system is in normal analyzing operation. In certain embodiments, only one of the at least one reaction cells occupies any station of the reaction cell washing arrangement at a time.

A variety of additional aspects will be set forth in the description that follows. These aspects can relate to individual features and to combinations of features. It is to be understood that both the foregoing general description and the following detailed description are exemplary and explanatory only and are not restrictive of the broad concepts upon which the embodiments disclosed herein are based.

### BRIEF DESCRIPTION OF THE DRAWINGS

Figure 1 is a schematic diagram illustrating an example instrument for analyzing biological specimens, according to the principles of the present disclosure;
Figure 2 is a schematic diagram illustrating a configurable wash process that is suitable for use in the example instrument of Figure 1, according to the principles of the present disclosure;
Figure 3 is a flow chart illustrating an example method of using the configurable wash process of Figure 2, according to the principles of the present disclosure;
Figure 4 is a time allocation chart illustrating a method of using the configurable wash process of Figure 2, according to the principles of the present disclosure;
Figure 5 is a schematic diagram illustrating a first example method for immunological analysis suitable for use in the example instrument of Figure 1, according to the principles of the present disclosure;
Figure 6 is a schematic diagram illustrating a second example method for immunological analysis suitable for use in the example instrument of Figure 1, according to the principles of the present disclosure;
Figure 7 is a perspective view, with a schematic component, of an example wash unit of the example instrument of Figure 1, according to the principles of the present disclosure;
Figure 8 is an elevation view of the wash unit of Figure 7;
Figure 9 is a top plan view of the wash unit of Figure 7;
Figure 10 is a perspective view of an example rotary portion of the example wash unit of Figure 7;
Figure 11 is a perspective view of two example linear portions of the example wash unit of Figure 7, in a first configuration;
Figure 12 is a perspective view of the two linear portions of Figure 11, in a second configuration, with additional schematic components;
Figure 13 is an elevation view of the two linear portions of Figure 11 in a configuration similar to the configuration of Figure 11;
Figure 14 is an elevation view of the two linear portions of Figure 11 in a configuration similar to the configuration of Figure 12;
Figure 15 is a perspective view of the two linear portions of Figure 11 in a configuration similar to the first configuration of Figure 11, but with additional probe assemblies shown;
Figure 16 is a perspective view of the two linear portions of Figure 11 in a configuration similar to the second configuration of Figure 12, but with the additional probe assemblies of Figure 15 shown;
Figure 17 is the elevation view of Figure 13, but with additional probe assemblies shown;
Figure 18 is the elevation view of Figure 14, but with the additional probe assemblies of Figure 17 shown;
Figure 19 is a cross-sectional elevation view of an example probe washing station of the example wash unit of Figure 7, shown in a first configuration and illustrated with a schematic probe washing flow-path;
Figure 20 is a cross-sectional elevation view of the example probe washing station of Figure 19, shown in a second configuration; and
Figure 21 is a cross-sectional elevation view similar to the cross-sectional elevation view of Figure 20, but with a probe partially inserted into a reaction vessel.

### DETAILED DESCRIPTION

Various embodiments will be described in detail with reference to the drawings, wherein like reference numerals represent like parts and assemblies throughout the several views. Reference to various embodiments does not limit the scope of the claims attached hereto. Additionally, any examples set forth in this specification are not intended to be limiting and merely set forth some of the many possible embodiments for the appended claims.

According to the principles of the present disclosure, a reaction cell washing arrangement (i.e., a wash unit) may wash a reaction cell in a sample analysis system. A variety of sample analysis systems with a variety of configurations are suitable for incorporating a reaction cell washing arrangement for a reaction cell, as will be understood by one of ordinary skill in the art.

According to the principles of the present disclosure, various probes P may be used to handle various fluids within a sample analysis system (e.g., a biological testing instrument). Fluids handled may include samples, specimens, reagents, chemicals, agents, rinses, particles, substrates, enzymes, unreacted substances, whole blood, serum, plasma, other blood components or fractions, immune complexes, etc. Assay reagents may include: wash buffers, buffer solutions, rinses, sample pretreatments, diluents, stains, dyes, substrates, antibody conjugates, enzymes or enzyme conjugates, nucleic acid conjugates, in reacted or unreacted states. Components of assay reagents typically include: water, buffers, chemicals, particles, substrates, enzymes, fixatives, preservatives, nucleic acids, antibodies, acids, bases, and mixtures thereof, in reacted or unreacted states. The probes P may aspirate and/or dispense the various fluids from and/or to various probe receiving stations PS within and/or adjacent to the sample analysis system. The probe receiving stations PS may be fixed or may be moveable.

One or more reaction cells (e.g., receptacles, reaction vessels, etc.) may be positioned at some or all of the probe receiving stations PS. The probes P may dispense and/or aspirate various fluids into and/or from the one or more reaction cells. The one or more reaction cells may include tubes, wells, cuvettes, etc. Each of the one or more reaction cells may be positioned at a single probe receiving station PS or may be moveable between probe receiving stations PS and/or other positions that are not probe receiving. Certain probes P may be specialized in dispensing fluids and may therefore only dispense fluids and not aspirate fluids. Likewise, certain probes P may be specialized in aspirating fluids and may therefore only aspirate fluids and not dispense fluids. Still other probes P may both aspirate and dispense fluids, as desired. To include probes P that may dispense only, aspirate only, and both dispense and aspirate, the conjunction "and/or" is used herein. Thus, mentioning a probe P for aspirating and/or dispensing fluid includes dispense only probes P, aspirate only probes P, and dispense and aspirate probes P.

Probes P may be actuated in a variety of ways suited to their particular functions in a particular sample analysis system. Certain probes P may be actuated along a single degree-of-freedom. The single degree-of-freedom may be a linear degree-of-freedom parallel to an axis of the probe P. Other probes P may be actuated along multiple degrees-of-freedom. Certain probes P may service a single location, while other probes P may service multiple locations. Certain probes P may receive fluid from a source (e.g., from a tank via a tube) and deliver (i.e., dispense) the fluid to one or more locations, while other probes P may remove (i.e., aspirate) the fluid from one or more locations and deliver fluid to a sink (e.g., to a tank via a tube). Still other probes P may aspirate one or more fluids from one or more locations and dispense one or more fluids to one or more locations and may thereby transfer one or more fluids between several locations. Various pumps, plumbing, valves, and conduits may be used to connect the probes P.

The locations serviced by the probes P may also vary according to their particular functions in a particular sample analysis system. For example, a probe P may aspirate and/or dispense fluid from and/or to various vessels, drains, supply reservoirs, waste collection reservoirs, tubes, sample tubes, wells, capped tubes, uncapped tubes, cuvettes, etc.

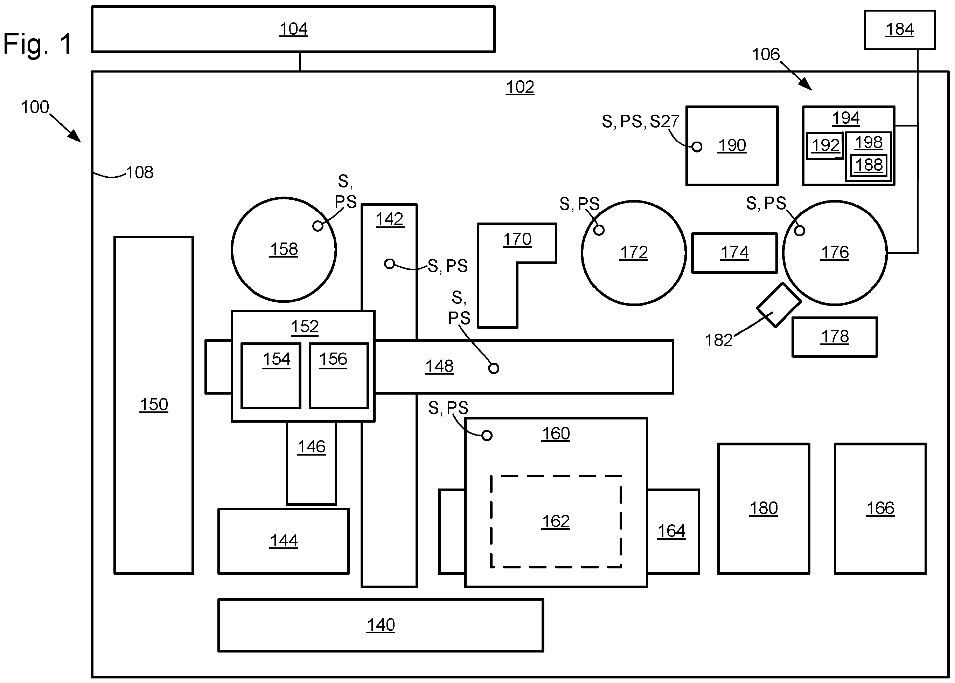

Turning now to Figure 1, a schematic diagram of an example instrument 100 (e.g., an immunoassay analyzer, a biological specimen analysis instrument, a sample analysis system, an immunoassay diagnostic system, an automated immunoassay diagnostic system, etc.) for analyzing a biological specimen is shown. In certain embodiments, the instrument 100 includes a substance preparation system 102, a preparation evaluation system 104, a substance evaluation system 106, and a frame 108 (e.g., a main frame). One or more containers are used with the systems of the instrument 100 and may include dispense tips and vessels. One or more container carriage devices may be provided in the instrument 100.

In certain embodiments, the example instrument 100 includes a computer 194 (i.e., a controller). The computer 194 may include memory 198 (e.g., non-transitory computer readable medium). The memory 198 may have data 188 stored thereon. The data 188 may include software, and various information, as is known in the art of such instruments. In certain embodiments, the data may include one or more assay protocol files (APFs). In certain embodiments, one or more assay protocol files (APFs) may be stored externally from the instrument 100 and may be accessed by the computer 194 (e.g., via a network). In certain embodiments, the computer 194 may include an evaluation processing device 192. In certain embodiments, the computer 194 may include a plurality of distributed computing devices and/or controllers.

Figure 1 schematically illustrates the example instrument 100. In the illustrated example, the instrument 100 is configured as an immunoassay analyzer. In certain embodiments, the substance preparation system 102 includes a sample supply board 140, a sample presentation unit 142, a reaction vessel feeder 144, a reaction vessel carriage unit 146, a sample transfer unit 148, a pipetting tip feeder 150, a sample pipetting device 152, a sample aliquot pipetting unit 154, a sample precise pipetting unit 156, a sample wheel 158, a reagent carriage unit 160, a reagent pipetting device 162, a reagent storage device 164, a reagent load device 166, a reaction vessel transfer unit 170 (i.e., an incubator transfer unit), an incubator 172, a reaction vessel transfer unit 174, a wash unit 176 (i.e., a wash wheel, a reaction wash unit, an assay wash area, an assay wash unit, etc.), a substrate pipetting device 178, a substrate load device 180, and a camera unit 182. In certain embodiments, the substance evaluation system 106 includes a light measurement device 190 and/or the evaluation processing device 192.

The sample presentation unit 142, the sample transfer unit 148, the sample wheel 158, the reaction vessel transfer unit 170, the incubator 172, the reaction vessel transfer unit 174, and the wash unit 176 may each include at least one carrier for transporting at least one sample vessel 220, 320 (e.g., a cuvette) between at least two stations S.

Certain embodiments of the substance evaluation system 106 are further associated with at least some operations performed by the reaction vessel transfer unit 170, the incubator 172, the reaction vessel transfer unit 174, the wash unit 176, and/or the substrate load device 180.

The frame 108 may provide a connecting structure that may hold some or all of the various systems and/or sub-systems of the instrument 100 with respect to each other. The frame 108 may further provide an interface (e.g., feet) to support the instrument 100 and to connect the instrument 100 to its operating environment (e.g., a lab, a building, etc.).

Certain systems of the example instrument 100 include probes P to process various liquids that include samples, specimens, reagents, chemicals, agents, rinses, particles, substrates, enzymes, unreacted substances, whole blood, serum, plasma, other blood components or fractions, immune complexes, urine, biological fluids, etc. Such systems may include the sample pipetting device 152, the sample aliquot pipetting unit 154 (i.e., the sample aliquot gantry), the sample precise pipetting unit 156 (i.e., the sample precision gantry), the reagent pipetting device 162, the wash unit 176 (i.e., the wash wheel), and the substrate pipetting device 178.

For example, the sample aliquot pipetting unit 154 operates to pipette an aliquot of sample from a sample tube located in the sample presentation unit 142 and dispense the aliquot of sample into a sample vessel on the sample wheel 158 with a probe P. For another example, the sample precise pipetting unit 156 operates to pipette the sample from a sample vessel located on the reagent carriage unit 160 with a probe P. Then, the sample precise pipetting unit 156 can dispense the sample to a reaction vessel with the probe P. In certain embodiments, the sample can be dispensed first to a dilution vessel with a probe P to create a sample dilution (for example, with buffer solution provided with a probe P by the reagent pipetting device 162) before being dispensed to a reaction vessel with a probe P. For still another example, the substrate pipetting device 178 operates to dispense a substrate 240 to a washed reaction vessel in the wash unit 176 with a probe P. One example of the substrate 240 is a chemiluminescent substrate for immunoassay enzyme reaction, such as *Lumi-Phos 530,* which can produce light and thereby provide detection corresponding to a quantity of analytes captured on magnetic particles. Another example of the substrate 240 includes those with features and/or characteristics described at WO 2018/006059 A1, titled Chemiluminescent Substrates, published 04 January 2018.

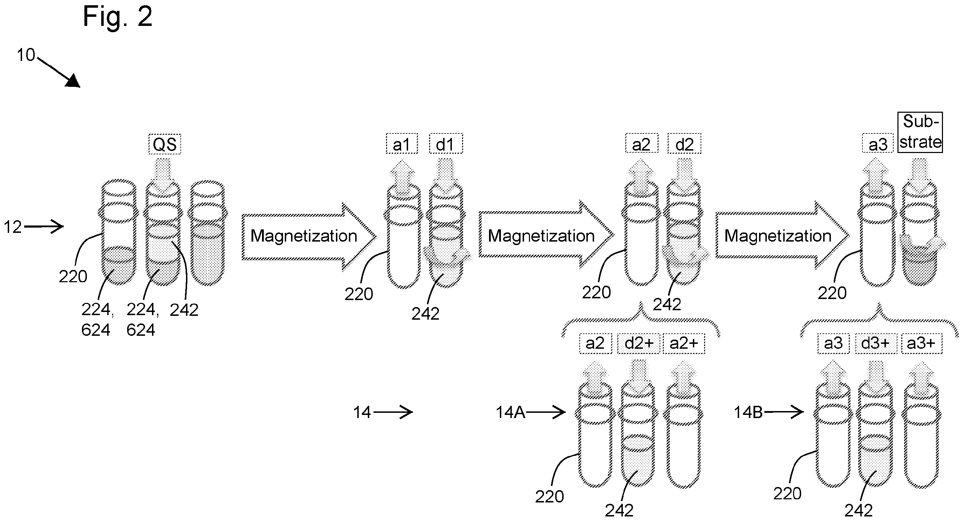

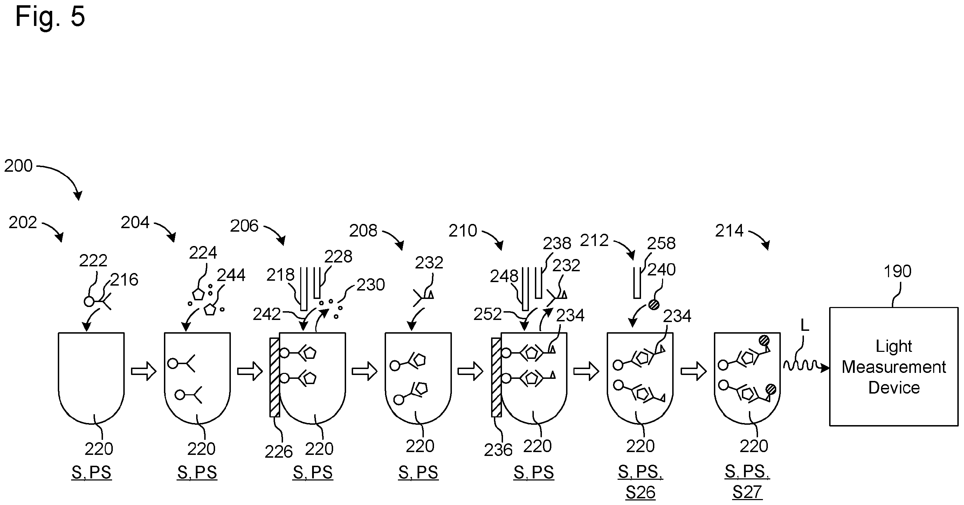

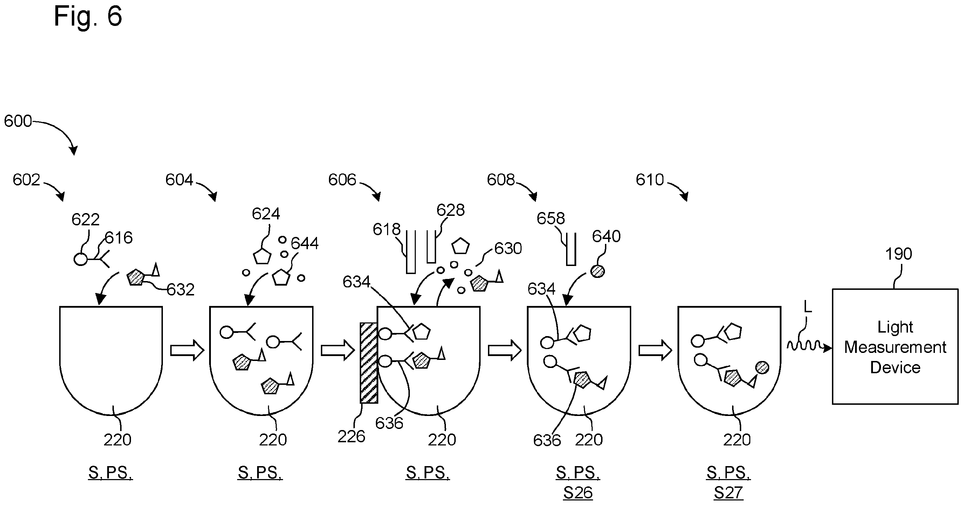

Turning now to Figure 2, a schematic diagram of an example configurable wash process 10 that is suitable for use in the example instrument 100 is illustrated, according to the principles of the present disclosure. As depicted, the configurable wash process 10 uses three probes QS, d1, and d2 that dispense clean buffer solution 242 (i.e., buffer fluid, wash buffer, rinse, etc.) into a vessel 220 (e.g., a reaction vessel, a container, a reaction cell, a cuvette, etc.) to wash away at least some unreacted components 230, 630 of a patient sample 224, 624 (see Figures 5 and 6) and/or at least some unreacted reagents 232 (e.g., free antigens, antibodies, unbound reactants, particles, and/or fluid, etc.). The configurable wash process 10 further uses three probes a1, a2, and a3 that aspirate (i.e., suck up) the at least some of the unreacted components 230, 630 of the patient sample 224, 624, some of the buffer solution 242, and/or the at least some of the unreacted reagents 232 from the vessel 220. Magnetization (e.g., using a magnetic collecting unit(s) 226, 236 or a magnetic collecting structure(s) 226, 236) may be used to retain desired components (e.g., immune complexes 234, 634, 636) within the vessel 220. In other embodiments, more than three probes or fewer than three probes may dispense the clean buffer solution 242 into the vessel 220. In other embodiments, more than three probes or fewer than three probes may aspirate the at least some of the unreacted components 230, 630 and/or unreacted reagents 232.

As illustrated at the upper row of Figure 2, a base number 12 of wash actions 206, 210, 606 (see Figures 5 and 6) may be performed if the three probes QS, d1, and d2 dispense buffer solution 242 once per vessel 220 and the three probes a1, a2, and a3 aspirate the at least some of the unreacted components 230, 630, some of the buffer solution 242, and/or the at least some of the unreacted reagents 232 once per vessel 220. The base number 12 of wash actions 206, 210, 606 may be preferred in certain assays. The base number 12 of wash actions 206, 210, 606 may be specified for certain assays in an assay protocol file 184 (i.e., an APF) corresponding to the assay.

However, certain other assays may prefer additional wash action(s) 206, 210, 606. An additional number of wash action(s) beyond the base number 12 of wash action(s) 206, 210, 606 may be specified for certain assays in an assay protocol file 184 (i.e., an APF). According to the principles of the present disclosure, certain probe(s) may be selectively used to dispense clean buffer solution 242 into the vessel 220 and aspirate the at least some of the unreacted components 230, 630 of the patient sample 224, 624, some of the buffer solution 242, and/or the at least some of the unreacted reagents 232 from the vessel 220 to perform the additional wash action(s) 206, 210, 606.

For example, as illustrated at the lower row of Figure 2, an additional number(s) 14 of wash actions 206, 210, 606 (see Figures 5 and 6) may be performed if at least some of the probes a2, a3 aspirate the at least some of the unreacted components 230, 630, some of the buffer solution 242, and/or the at least some of the unreacted reagents 232 from the vessel 220 and further dispense clean buffer solution 242 into the vessel 220 and again aspirate the at least some of the unreacted components 230, 630 of the patient sample 224, 624, some of the buffer solution 242, and/or the at least some of the unreacted reagents 232 from the vessel 220 to perform the additional wash action(s) 206, 210, 606.

As further illustrated at the lower row of Figure 2, the additional number(s) 14 of wash actions 206, 210, 606 (see Figures 5 and 6) may include one, a plurality, or all of the potential additional number(s) 14 of wash actions 206, 210, 606. In particular, either one 14A or 14B or both 14A and 14B of the potential additional number(s) 14 of wash actions 206, 210, 606 may be used. (If the base number 12 of wash actions 206, 210, 606 (see Figures 5 and 6) is performed, then no additional number(s) 14 of wash actions 206, 210, 606 is performed).

Assays using substrates including features and/or characteristics described at WO 2018/006059 A1, introduced above, may benefit from one or more additional number(s) 14 of wash actions 206, 210, 606. Sandwich assays may benefit from one or more additional number(s) 14 of wash actions 206, 210. Sandwich assays using substrates including features and/or characteristics described at WO 2018/006059 A1 may benefit from one or more additional number(s) 14 of wash actions 206, 210.

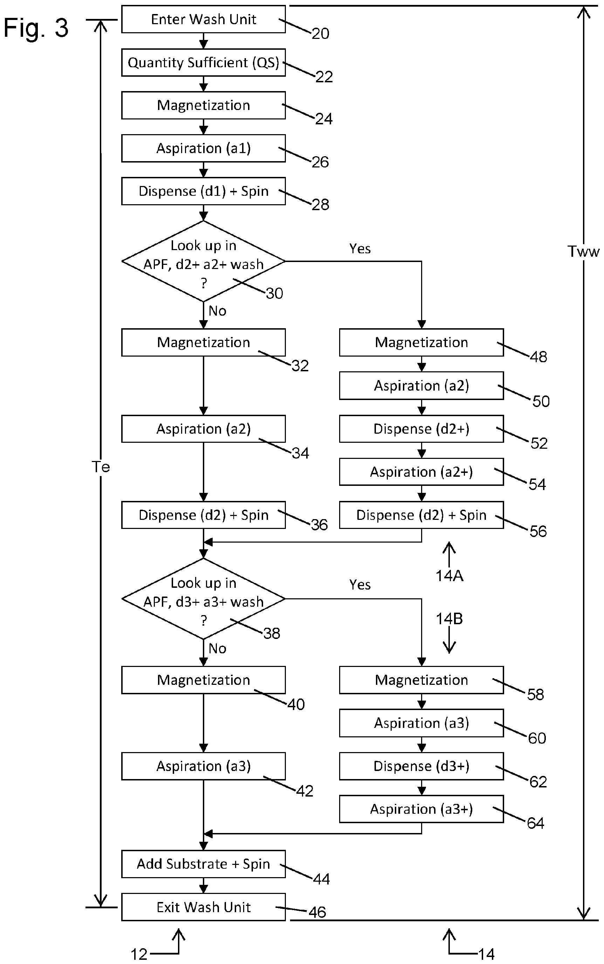

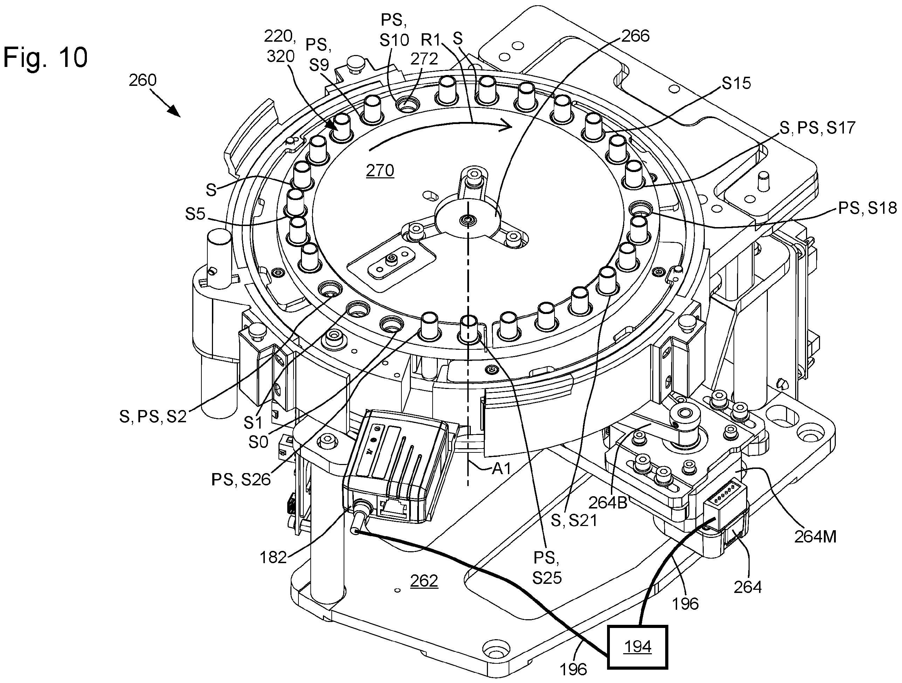

Turning now to Figure 3, a flow chart for an example method of using the configurable wash process 10 is illustrated, according to the principles of the present disclosure. The illustrated method begins at step 20 where a vessel (e.g., a reaction cell 220, 320) enters a wash unit (e.g., the washing arrangement 176). In the example wash unit 176, the vessel 220 enters at station S1 (see Figure 10). Upon a predetermined cycle time (e.g., 8 seconds) of the example instrument 100 passing, the wash unit 176 advances one pitch and thereby transfers the vessel 220 to station S2 and thereby starts step 22.

At step 22, the vessel 220 receives buffer solution 242 from a probe P (e.g., probe assembly 288A), schematically illustrated at Figure 2 as probe QS. Upon another cycle time passing, the wash unit 176 advances one pitch and thereby transfers the vessel 220 to station S3 and thereby starts step 24. At step 24, a magnetic field is applied to the vessel 220 for reasons described herein. The example washing arrangement 176 has 6 stations S3-S8 that apply a magnetic field, and step 24 lasts 6 cycle times (e.g., 48 seconds) passing through stations S3-S8. The wash unit 176 then advances one pitch and thereby transfers the vessel 220 to station S9 and thereby starts step 26.

At step 26, a probe P (e.g., probe assembly 298A), schematically illustrated at Figure 2 as probe a1, aspirates fluid from the vessel 220 while a magnetic field is applied. Upon another cycle time passing, the wash unit 176 advances one pitch and thereby transfers the vessel 220 to station S10 and thereby starts step 28.

At step 28, the vessel 220 receives buffer solution 242 from a probe P (e.g., probe assembly 288B), schematically illustrated at Figure 2 as probe d1, and further receives spin mixing. Upon another cycle time passing, the wash unit 176 advances one pitch and thereby transfers the vessel 220 to station S11 and thereby starts either step 32 or 48. In the flow chart of Figure 3, information is looked up in an APF for the assay in process and used at decision point 30. This information may be looked up before or at the start of steps 32 or 48.

If the result of decision point 30 is "No", then at step 32 a magnetic field is applied to the vessel 220 for reasons described herein. The example washing arrangement 176 has 6 stations S1 1-S16 that apply a magnetic field, and step 32 lasts 6 cycle times (e.g., 48 seconds) passing through stations S 11-S 16. The wash unit 176 then advances one pitch and thereby transfers the vessel 220 to station S17 and thereby starts step 34.

At step 34, a probe P (e.g., probe assembly 298B), schematically illustrated at Figure 2 as probe a2, aspirates fluid from the vessel 220 while a magnetic field is applied. Upon another cycle time passing, the wash unit 176 advances one pitch and thereby transfers the vessel 220 to station S18 and thereby starts step 36.

At step 36, the vessel 220 receives buffer solution 242 from a probe P (e.g., probe assembly 288C), schematically illustrated at Figure 2 as probe d2, and further receives spin mixing. Upon another cycle time passing, the wash unit 176 advances one pitch and thereby transfers the vessel 220 to station S19 and thereby starts either step 40 or 58. In the flow chart of Figure 3, information is looked up in the APF for the assay in process and used at decision point 38. This information may be looked up before or at the start of steps 40 or 58.

If the result of decision point 30 is "Yes", then at step 48 a magnetic field is applied to the vessel 220 for reasons described herein. As mentioned above, the example washing arrangement 176 has 6 stations S11-S16 that apply a magnetic field, and step 32 lasts 6 cycle times (e.g., 48 seconds) passing through stations S11-S16. The wash unit 176 then advances one pitch and thereby transfers the vessel 220 to station S17 and thereby starts step 50.

At step 50, a probe P (e.g., probe assembly 298B), schematically illustrated at Figure 2 as probe a2, aspirates fluid from the vessel 220 while a magnetic field is applied. At step 52, the probe P (e.g., the probe assembly 298B) further dispenses buffer solution 242 into the vessel 220 while a magnetic field is applied. At step 54, the probe P (e.g., the probe assembly 298B) further aspirates fluid from the vessel 220 while a magnetic field is applied. By further dispensing (d2+ at step 52) and aspirating (a2+ at step 54), the additional wash action 14A is performed at steps 52 and 54. Upon another cycle time passing (as steps 50, 52, and 54 are all completed), the wash unit 176 advances one pitch and thereby transfers the vessel 220 to station S18 and thereby starts step 56.

At step 56, the vessel 220 receives buffer solution 242 from a probe P (e.g., probe assembly 288C), schematically illustrated at Figure 2 as probe d2, and further receives spin mixing. Upon another cycle time passing, the wash unit 176 advances one pitch and thereby transfers the vessel 220 to station S19 and thereby starts either step 40 or 58. In the flow chart of Figure 3, information is looked up in the APF for the assay in process and used at decision point 38. This information may be looked up before or at the start of steps 40 or 58.

If the result of decision point 38 is "No", then at step 40 a magnetic field is applied to the vessel 220 for reasons described herein. The example washing arrangement 176 has 6 stations S19-S24 that apply a magnetic field, and step 40 lasts 6 cycle times (e.g., 48 seconds) passing through stations S19-S24. The wash unit 176 then advances one pitch and thereby transfers the vessel 220 to station S25 and thereby starts step 42.

At step 42, a probe P (e.g., probe assembly 298C), schematically illustrated at Figure 2 as probe a3, aspirates fluid from the vessel 220 while a magnetic field is applied. Upon another cycle time passing, the wash unit 176 advances one pitch and thereby transfers the vessel 220 to station S26 and thereby starts step 44.

If the result of decision point 38 is "Yes", then at step 58 a magnetic field is applied to the vessel 220 for reasons described herein. The example washing arrangement 176 has 6 stations S19-S24 that apply a magnetic field, and step 58 lasts 6 cycle times (e.g., 48 seconds) passing through stations S19-S24. The wash unit 176 then advances one pitch and thereby transfers the vessel 220 to station S25 and thereby starts step 60.

At step 60, a probe P (e.g., probe assembly 298C), schematically illustrated at Figure 2 as probe a3, aspirates fluid from the vessel 220 while a magnetic field is applied. At step 62, the probe P (e.g., the probe assembly 298C) further dispenses buffer solution 242 into the vessel 220 while a magnetic field is applied. At step 64, the probe P (e.g., the probe assembly 298C) further aspirates fluid from the vessel 220 while a magnetic field is applied. By further dispensing (d3+ at step 62) and aspirating (a3+ at step 64), the additional wash action 14B is performed at steps 62 and 64. Upon another cycle time passing (as steps 60, 62, and 64 are all completed), the wash unit 176 advances one pitch and thereby transfers the vessel 220 to station S26 and thereby starts step 44.

At step 44, the vessel 220 receives substrate 240 from a probe P (e.g., probe assembly 288D), schematically illustrated at Figure 2 as probe "Substrate", and further receives spin mixing. Upon another cycle time passing, the wash unit 176 advances one pitch and thereby transfers the vessel 220 to station S0. No function occurs at station S0, other than the transport of the vessel 220. Upon another cycle time passing, the wash unit 176 advances one pitch and thereby transfers the vessel 220 to station S1 and thereby starts step 46. At step 46, the vessel 220 is removed from the wash unit (e.g., the washing arrangement 176).

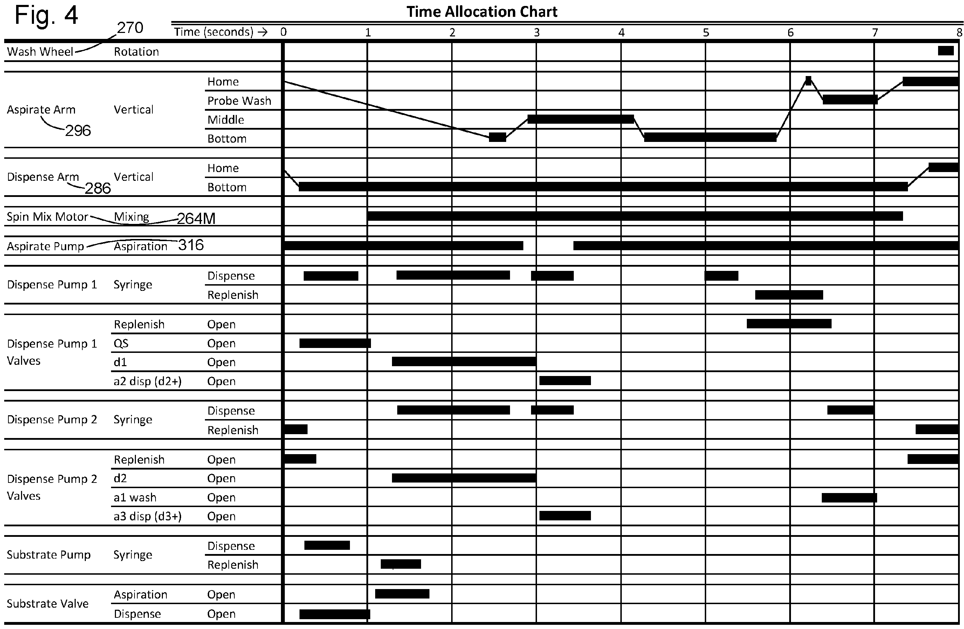

Turning now to Figure 4, a time allocation chart for a method of using the configurable wash process 10 is illustrated, according to the principles of the present disclosure. As mentioned above, in the example embodiment, the instrument 100 operates on a predetermined cycle time (e.g., 8 seconds). Figures 2-4 illustrate a method of configuring the wash unit 176 to provide a base number 12 of wash actions 206, 210, 606 equal to three wash actions and optionally providing one (14A or 14B) or two (14A and 14B) of additional number(s) 14 of wash actions 206, 210, 606. Whether only the base number 12 of three wash actions 206, 210, 606 is used; whether one (14A or 14B) additional number 14 of wash actions 206, 210, 606 is used; or whether two (14A and 14B) of additional numbers 14 of wash actions 206, 210, 606 are used, the total elapsed time Te for the vessel 220 in the wash unit 176 is the same. In the example wash unit 176, there are 27 stations S and a predetermined cycle time of 8 seconds, giving 27 stations times 8 seconds per station or about 216 seconds of total elapsed time Te for the vessel 220 in the wash unit 176. (There may be a few seconds where no vessel 220 can occupy station S 1 due to in and out travel time. Thus, the total elapsed time Te will be slightly less than 216 seconds.)

Figure 3 schematically illustrates the total elapsed time Te that the vessel 220 spends in the wash unit 176 and further illustrates that the total elapsed time Te for the vessel 220 in the wash unit 176 is always the same (regardless of the number of washes). Figure 3 also illustrates the various timed sequences that may be used, depending on the APF file, and that each of the various timed sequences have the same total elapsed time Te. Thus, each of the timed sequences are a part of a predetermined timed sequence Tww.

Turning now to Figure 5, a schematic diagram that illustrates an example method 200 (i.e., an assay, a sandwich assay, an assay type, etc.) for immunological analysis using the example instrument 100 will now be described in detail. In certain embodiments, the method 200 includes operations 202, 204, 206, 208, 210, 212, and 214. In certain embodiments, at least some of the operations in the method 200 are performed by the substance preparation system 102, the preparation evaluation system 104, and/or the substance evaluation system 106 of the example instrument 100. As illustrated at Figure 5, the analyte is an antigen that is captured by an antibody. In other embodiments, the analyte may be an antibody that is captured by an antigen. According to the principles of the present disclosure, various combinations of anti-analytes and analytes, such as these, are suitable for use on the apparatuses and with the methods and processes disclosed herein.

At operation 202, a vessel 220 (e.g., a reaction vessel, a container, a reaction cell, a cuvette, etc.) may be transported to a predetermined position S (e.g., a station), and a first reagent 216, including antibodies and magnetic particles 222, may be dispensed into the vessel 220 by a probe P. For purposes of this disclosure, the term "fluid" includes fluids with particles (e.g., suspended particles) such as the first reagent 216 with magnetic particles 222.

At operation 204, a sample or specimen 224 (e.g., a fluid, a sample, a patient sample, or specimen suspended or mixed in a fluid, etc.) is dispensed into the vessel 220 by a probe P. In certain embodiments, the sample pipetting device 152, aspirates, with a probe P, the sample 224 from a sample vessel that has been transported to a predetermined position S. Once the sample 224 is dispensed into the vessel 220, the vessel 220 may be subjected to mixing and/or incubating, if required, so as to produce magnetic particle carriers each formed of an antigen in the sample 224 and the magnetic particle 222 bonded together.

At operation 206, the vessel 220 may be subjected to a first cleaning process in which the magnetic particle carriers are magnetically collected by a magnetic collecting unit 226 (i.e., a magnetic collecting structure). A bound-free separation is carried out by a bound-free cleaning dispense nozzle 228 (i.e., a probe P) dispensing a rinsing fluid 242 and by a bound-free cleaning aspiration nozzle 218 (i.e., a probe P) aspirating uncollected fluid components. The aspiration nozzle 218 may be washed with a probe washer 470 before and/or after the aspirating. The bound-free separation may include a series of dispensing the rinsing fluid 242 and aspirating the uncollected fluid components. As a result, an unreacted substance 230 or substances 230 (e.g., unbound reactants, particles, and/or fluid, etc.) in the vessel 220 is removed (e.g., rinsed away) by the bound-free cleaning aspiration nozzle 218.

At operation 208, a second reagent 232, such as a labeling reagent including a labeled antibody and/or a fluid, may be dispensed into the vessel 220 by a probe P. As a result, immune complexes 234, each formed of the magnetic particle carrier and the labeled antibody 232 bonded together, are produced.

At operation 210, a second bound-free cleaning process is performed to magnetically collect the magnetic particle carriers and thereby collect the immune complexes 234 by a magnetic collecting structure 236. Further, a bound-free separation, similar to or the same as that mentioned above, is performed by a bound-free cleaning dispense nozzle 238 (i.e., a probe P) dispensing a rinsing fluid 252 and by a bound-free cleaning aspiration nozzle 248 (i.e., a probe P) aspirating the uncollected fluid components. As a result, any labeled antibodies 232 that are not bonded with the carrier of the magnetic particles 222 are removed from the vessel 220 by the bound-free cleaning aspiration nozzle 248.

At operation 212, a substrate 240 including an enzyme and/or a fluid is dispensed into the vessel 220 by a substrate nozzle 258 (i.e., a probe P), for example at station S26 of the wash unit 176, describe in detail herein. The contents of the vessel 220 are then mixed. After a certain reaction time necessary for the enzyme reaction passes (e.g., in the incubator 172), the vessel 220 is transported to a photometric system, such as to a station S27 of the light measurement device 190.

At operation 214, the enzyme 240 and the immune complex 234 are bonded together through the substrate 240 reactions with the enzyme on the labeled antibody 232, and light L is emitted and measured by a photometric system, such as the light measurement device 190. The light measurement device 190 operates to calculate an amount of antigen that is included in the specimen 224, according to the quantity of light L measured.

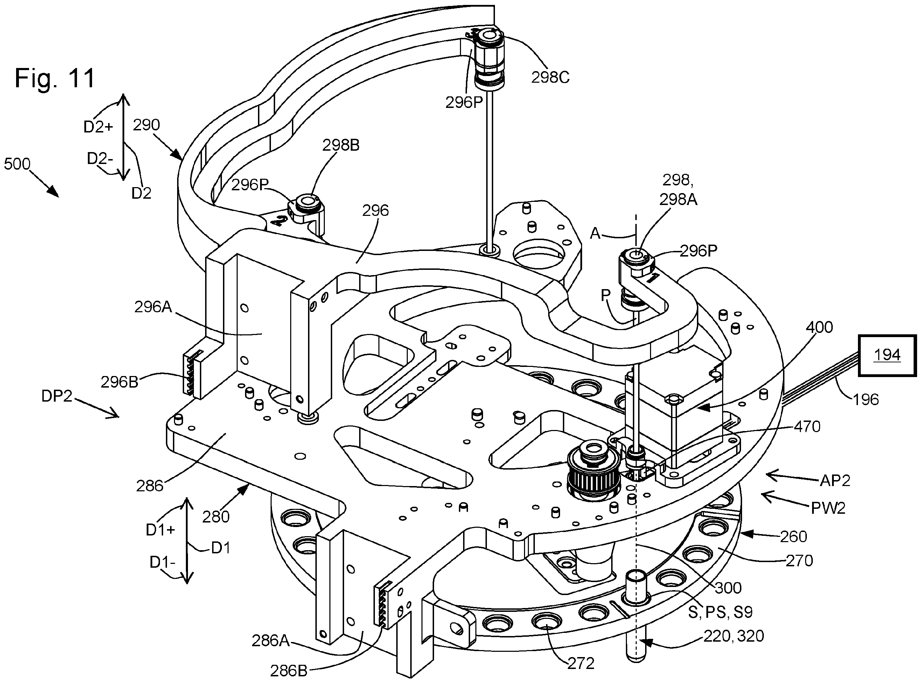

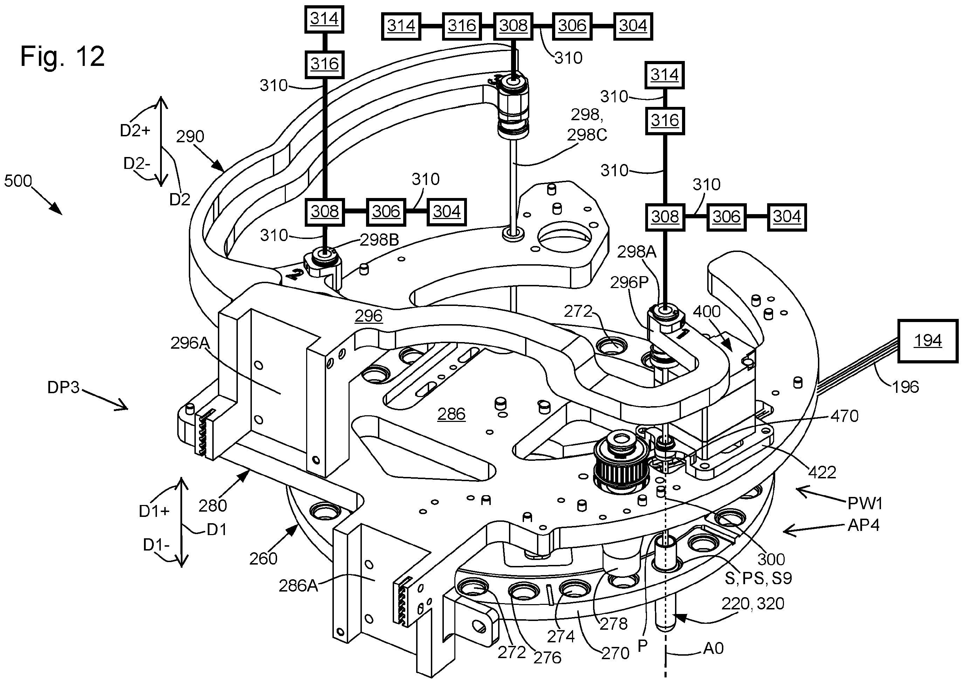

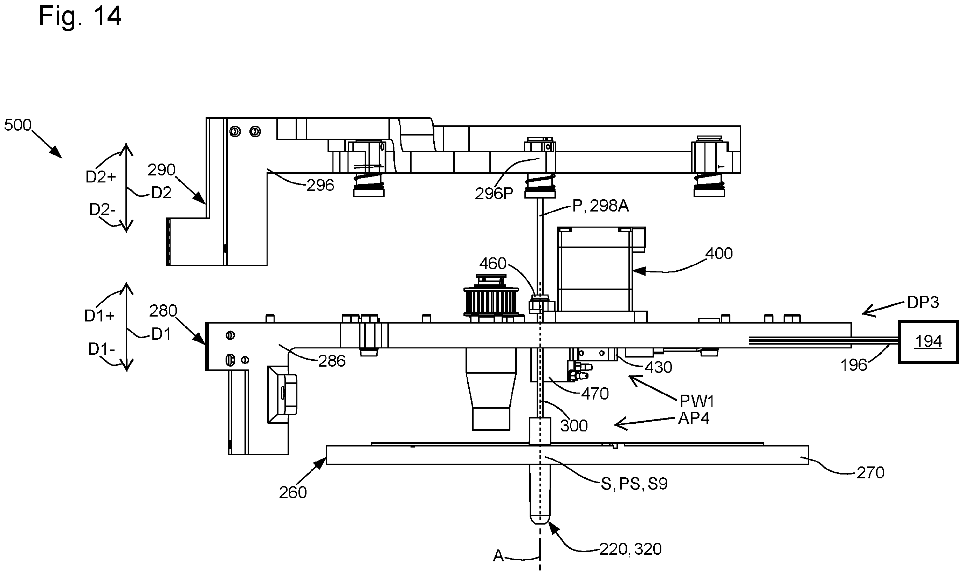

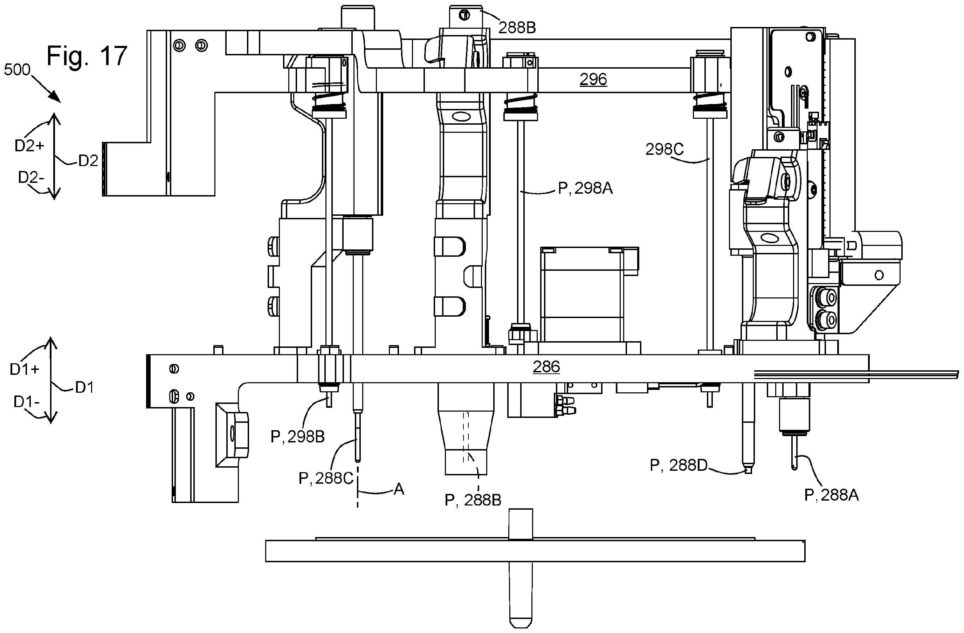

The vessel 220 may include a revolved form that is axisymmetric about an axis A0 (see Figure 12). The probe receiving station PS (see Figure 11) may hold the vessel 220 at a predetermined location and thereby hold the axis A0 of the vessel 220 at a predetermined position. The probe P may include a revolved form that is axisymmetric about an axis A (see Figures 11, 14, and 17). The probe P may define the axis A. The probe receiving station PS may define the axis A0. The probe P may be aligned with the corresponding probe receiving station PS when the axes A and A0 are aligned within an acceptable tolerance.

Turning now to Figure 6, a schematic diagram that illustrates an example method 600 (i.e., an assay, a competitive assay, an assay type, etc.) for immunological analysis using the example instrument 100 will now be described. For analytes that are too small (approximately 200 - 8,000 Daltons (Da)) to accommodate two antibodies to bind at the same time, a competitive assay format may be used.

As the configurable washing arrangement 176 may employ a configurable number of wash actions, a wide variety of assays (e.g., sandwich assays and competitive assays) may be processed by the same instrument 100.

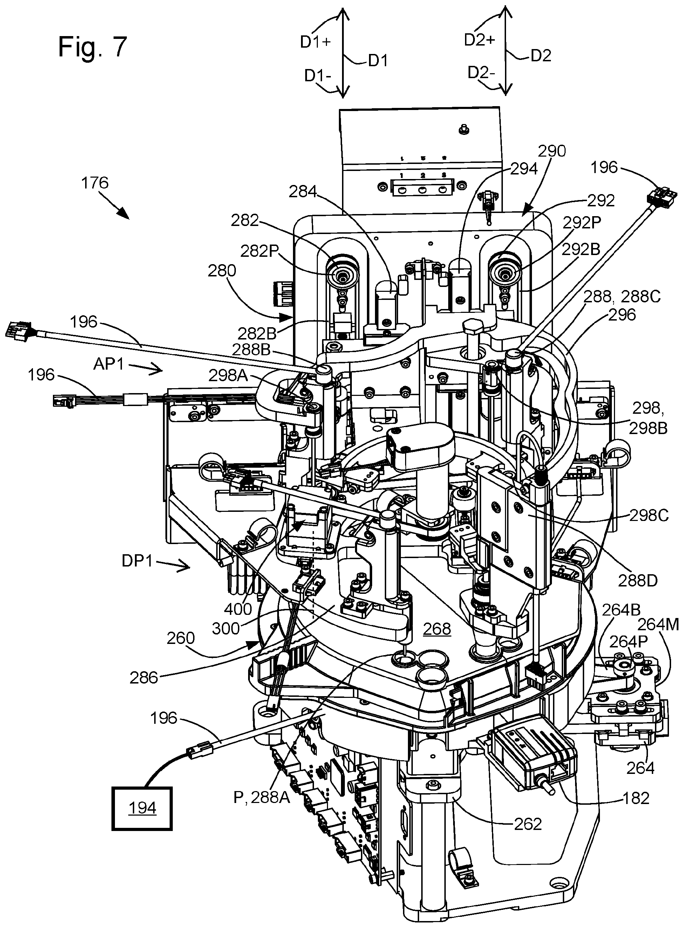

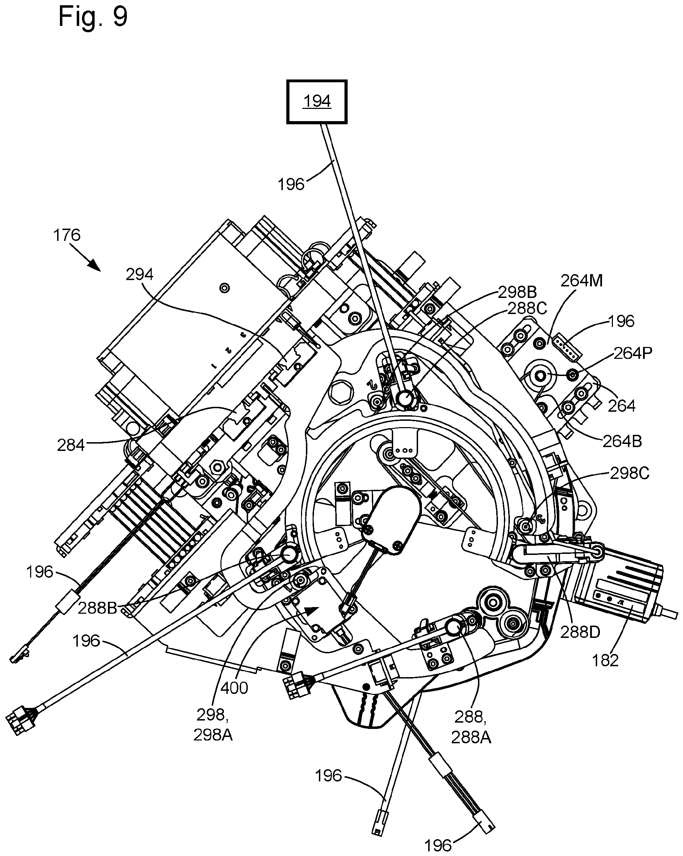

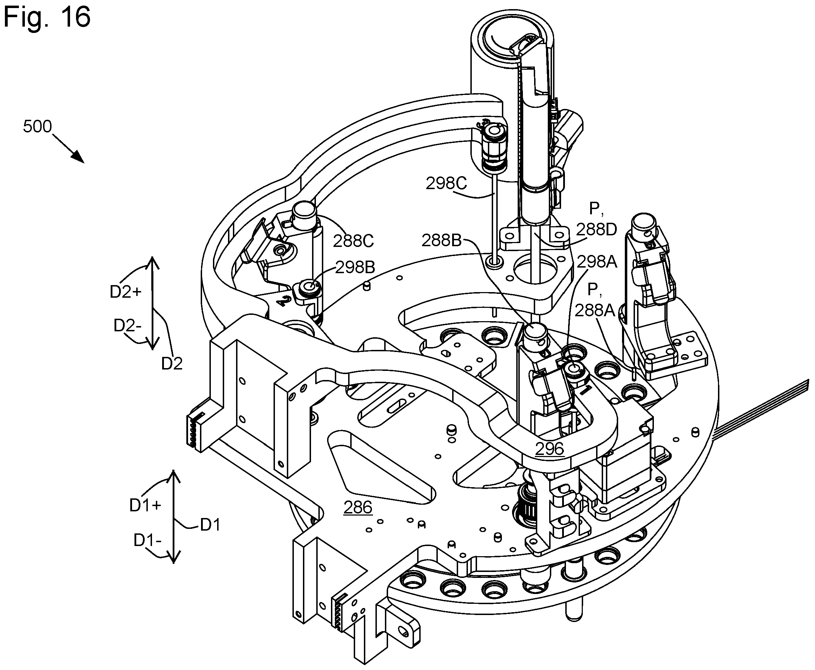

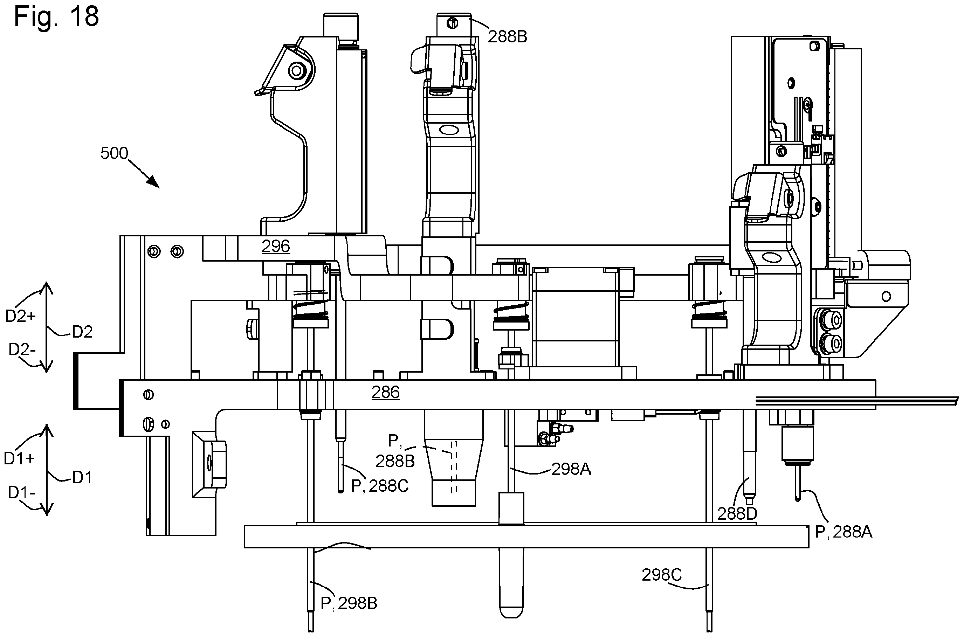

Turning now to Figures 7-9, the example wash unit 176 will be further described. An example carrier arrangement 260 of the wash unit 176 is further illustrated at Figure 10. An example first probe arrangement 280 and an example second probe arrangement 290 of the wash unit 176 are further illustrated at Figures 11-18. The wash unit 176 is configured to process biological samples according to operations 206, 210, and 212, described above with reference to Figure 5 and operations 606 and 608 with reference to Figure 6. The wash unit 176 may be further configured to carry out additional operations.

As depicted, the first probe arrangement 280 and the second probe arrangement 290 are included in an example fluid handling system 500 of the wash unit 176, according to the principles of the present disclosure. The fluid handling system 500, including the probe arrangements 280 and 290, is tailored to the configuration of the wash unit 176 and interfaces with the carrier arrangement 260 of the wash unit 176.

Turning to Figure 10, the carrier arrangement 260 of the wash unit 176 will be described in detail. The carrier arrangement 260 includes a carrier 270 (e.g., a carrier wheel, a carrier disk, a carrier ring, etc.). The carrier 270 includes a plurality of holders 272 (e.g., holes, etc.). As depicted, the carrier 270 includes 27 example holders 272. In other embodiments, the carrier 270 may include less than or more than 27 holders 272. As illustrated at Figure 12, each of the holders 272 includes an example through-hole 274, and an example counter-bore 276. The holders 272 are each configured to receive an example vessel 320 (i.e., a sample vessel, a reaction vessel, a cuvette, etc.). In the example embodiment, the holder 272 and the vessel 320 are each axisymmetric and are axisymmetric with each other, when mated. The vessel 320 may include a revolved form that is axisymmetric about the axis A0. The probe receiving station PS may hold the vessel 320 at a predetermined location and thereby hold the axis A0 of the vessel 320 at a predetermined position. The probe P may include a revolved form that is axisymmetric about the axis A.

In the example depicted, the wash unit 176 defines 27 stations S about which the carrier 270 moves the holders 272 between. In particular, the carrier 272 rotates about an axis A1 and thereby moves the holders 272 from station S to station S about a rotational displacement R1. In the example embodiment, the carrier 270 is indexed 13 1/3 degrees per cycle and thereby advances each of the 27 holders 272 one station forward per cycle. In the depicted embodiment, the carrier 270 is rotary. In other embodiments, other carriers may be non-rotary. In the example embodiment, the carrier 270 includes a single holder 272 at each station at one time. In other embodiments, other carriers may include multiple holders per station at the same time. In the example embodiment, the carrier 272 moves about the axis A1 and thereby moves about a single degree-of-freedom. In the example embodiment, the carrier 272 rotates about the axis A1 and thereby rotates about a single rotational degree-of-freedom. In the example embodiment, the axis A1 is vertical.

At Figure 10, the stations S are labeled with respect to the carrier 270 at a given position. The stations S remain at the positions indicated as the carrier 270 is indexed. The stations S are thus fixed to a frame 262 of the carrier arrangement 260 as the carrier 270 indexes. At Figure 10, the stations S are designated with a station number given by "S" followed by the station number. Not all stations S are labeled, but can be determined by counting between the labeled stations S.

A description of the various stations S will now be given. Station S0 is a no-function station, but may transfer the vessel 320 between neighboring stations S (e.g., stations S26 and S1). Station S1 is an entrance/exit station. The vessel 320 is introduced to one of the holders 272 of the carrier 270 at station S1 (i.e., whichever holder 272 happens to be at station S1 when the particular vessel 320 arrives). From station S1, the vessel 320 is indexed around to the other stations S and eventually returns to the station S1, where it is removed from the holder 272 of the carrier 270.

In the context of Figures 1 and 10, the reaction vessel transfer unit 174 may remove a washed vessel 320 from the station S1 every cycle and replace it with a vessel 320 to be washed. Certain cycles (e.g., an idle cycle with no activity scheduled) may not necessarily transfer a vessel 320 into one of the holders 272 that is currently at the station S1, thereby leaving an unfilled holder 272. When such an unfilled holder 272 returns to the station S1, there will be no vessel 320 to remove. Figure 10 illustrates such unfilled holders 272 at stations S0, S1, S2, S10, and S18. The empty holders 272 also advance from station S to station S as the carrier 270 advances.

At station S2, the vessel 320, if present, receives fluid from a probe P of a probe assembly 288A (see Figures 7-9 and 15-18). The probe assembly 288A may be a quantity sufficient probe assembly and thereby dispense fluid to bring the fluid level in the vessel 320 up to a predetermined level, even though existing fluid in the vessel 320 may vary. In the example embodiment, stations S3-S8 are magnetic stations, similar to the stations S at operations 206 and 210 at Figure 5 and operation 606 at Figure 6.

Station S9 receives a probe P of a probe assembly 298A (see Figures 7-9 and 11-21), and the probe P thereby aspirates fluid from within the vessel 320. The station S9 is also a magnetic station, like the stations S3-S8.

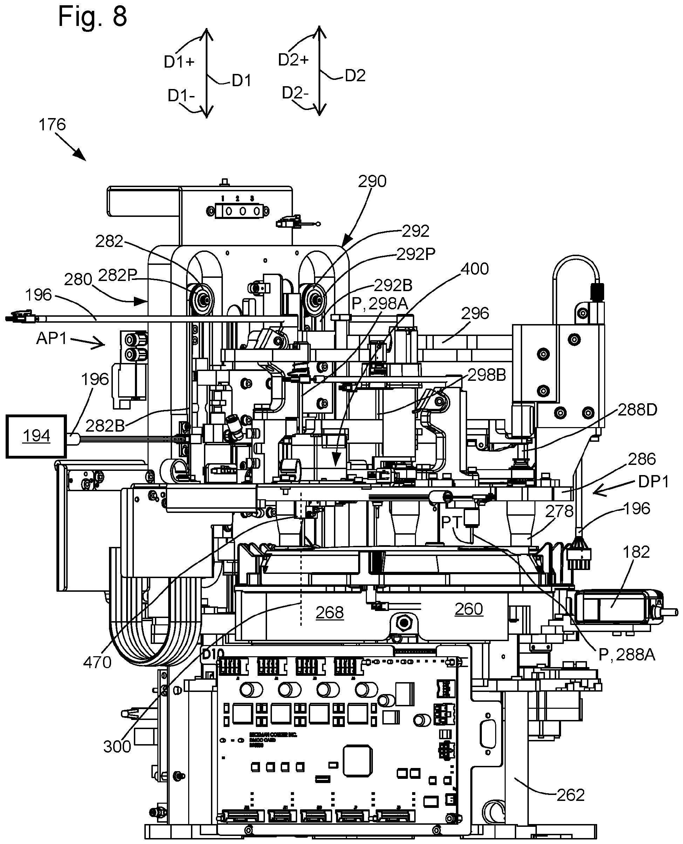

Station S10 receives a probe P of a probe assembly 288B (see Figures 7, 9, and 15-18) which dispenses fluid into the vessel 320. The station S10 further includes a spin-mixer 278 (see Figures 8, 12, and 13) which may be used to spin-mix contents within the vessel 320. Stations S11-S16 are magnetic stations similar to the magnetic stations S3-S9.

Station S17 receives a probe P of probe assembly 298B (see Figures 7-9, 11, 12, and 15-18) which aspirates fluid from the vessel 320. The station S17 is also a magnetic station, like the magnetic stations S3-S9 and S11-S16. The probe assembly 298B may further dispense and aspirate at station S17, as mentioned above and illustrated at Figures 3 and 4.

Station S18 receives a probe P of a probe assembly 288C (see Figures 7, 9, and 15-18) which dispenses fluid into the vessel 320. Like the station S10, the station S18 includes a spin-mixer 278 and thereby spin-mixes the contents of the vessel 320. Stations S19-S24 are magnetic stations, like magnetic stations S3-S9 and S11-S17.

Station S25 receives a probe P of a probe assembly 298C (see Figures 7, 9, 11, 12, and 15-18) and thereby aspirates fluid from the vessel 320. Station S25 is also a magnetic station, like magnetic stations S3-S9, S11-S17, and S19-S24. The probe assembly 298C may further dispense and aspirate at station S25, as mentioned above and illustrated at Figures 3 and 4.

Station S26 receives a probe P of a probe assembly 288D (see Figures 7-9 and 15-18) which dispenses a substrate 240 into the vessel 320, similar to that shown at station S26 of Figures 5 and 6. Like the stations S10 and S18, the station S26 includes a spin-mixer 278 and thereby spin-mixes the contents of the vessel 320. From the station S26, the carrier 270 advances the vessel 320 to the station S0. As mentioned above, no function occurs at station S0, other than the transport of the vessel 320.

As mentioned above, upon the carrier 270 indexing the vessel 320 from the station S0 to the station S1, the vessel 320 is ready to be removed from the carrier 270. In particular, the reaction vessel transfer unit 174 may retrieve the vessel 320 from the station S1 of the carrier arrangement 260 of the wash unit 176 and bring the vessel 320 to a station S of the incubator 172. Upon incubation being complete, the vessel 320 may be transferred to the light measurement device 190. In particular, the vessel 320 may be transferred to station S27 of the light measurement device 190. Figures 5 and 6 schematically illustrates light L being emitted from the fluid at station S27 and being measured by the light measurement device 190.

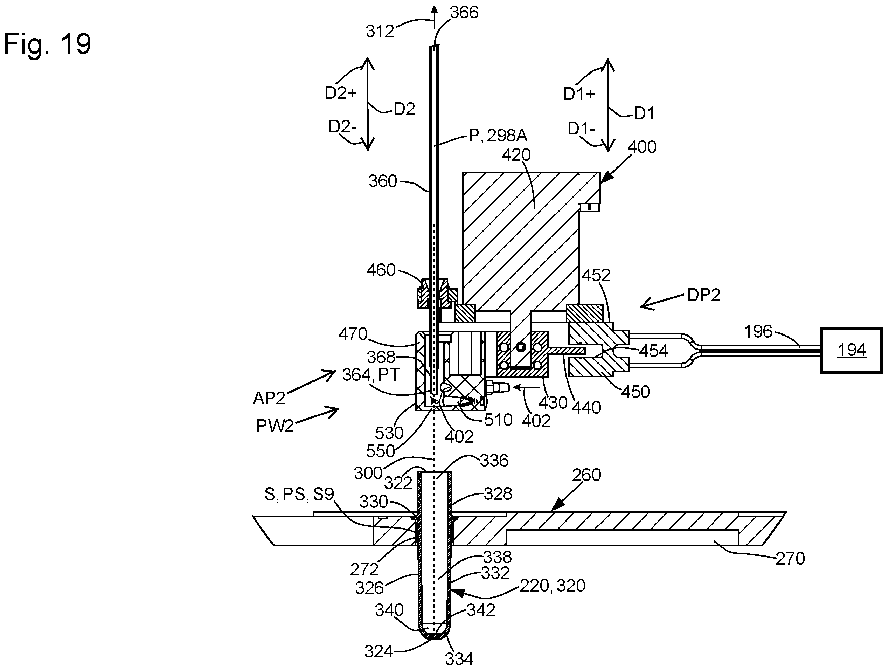

Turning now to Figures 12 and 19, the interface between the example vessel 320 and the holder 272 will now be described in detail. As illustrated at Figure 12, each of the holders 272 includes a through hole 274 that extends through the carrier 270. At a top side of the through hold 274, a counter bore 276 into the carrier 270 provides a recess. The through hole 274 and the counter bore 276 are axisymmetric with each other.

Turning now to Figure 19, the example vessel 320 will be described in detail. The example vessel 320 extends between a first end 322 and a second end 324. The second end 324 is rounded. The example vessel 320 further includes an exterior 326. The exterior 326 includes a first exterior portion 328 adjacent to the first end 322. The exterior 326 further includes a flange portion 330 adjacent to the first exterior portion 328 but opposite the first end 322 about the first exterior portion 328. The exterior 326 further includes a second exterior portion 332. The second exterior portion 332 is adjacent the flange portion 330. The exterior 326 further includes a third exterior portion 334 adjacent the second exterior portion 332 and adjacent the second end 324 opposite the second exterior portion 332. The third exterior portion 334 is rounded adjacent the second end 324. At the first end 322, the example vessel 320 includes an opening 336. An interior 338 of the example vessel 320 may be accessed via the opening 336. The interior 338 includes a bottom portion 340. The bottom portion 340 includes a bottom 342 of the interior 338.

As mentioned above, the example vessel 320 is substantially axisymmetric. The first exterior portion 328, the second exterior portion 332, and the interior 338, excluding the bottom portion 340, are substantially cylindrical, but may include draft for molding purposes and/or other purposes. When inserting the example vessel 320 into the holder 272, the rounded third exterior portion 334 may assist in guiding the vessel 320 into the holder 272. Upon further insertion of the example vessel 320 into the holder 272, the flange portion 330 of the vessel 320 abuts a bottom of the counter bore 276 of the holder 272 and thereby seats the vessel 320 in the holder 272. A small radial clearance may be present between the second exterior portion 332 and the through hole 274 and thereby allows the vessel 320 to spin within the holder 272 when spin-mixing occurs.

Turning again to Figure 10, the carrier arrangement 260 of the wash unit 176 will be described in further detail. The carrier arrangement 260 is attached to the wash unit 176. In particular, the frame 262 of the carrier arrangement 260 is fixedly attached to a frame of the wash unit 176. The rotational movement of the carrier 270 is accomplished by a drive 264 (see Figures 7, 9, and 10). The drive 264 includes a motor 264M, a pulley 264P, and a belt 264B. The carrier 270 rotates about the axis A1 of a hub 266. The belt 264B engages a pulley (not shown) of the hub 266. Thus, when the motor 264M rotates, the carrier 270 also rotates. The motor 264M is connected to the computer 194 by a wiring harness 196. The motor 264M may further be connected to a power supply by the wiring harness 196. The computer 194 thereby controls rotation of the motor 264M and thereby further controls the rotational movement of the carrier 270. As illustrated at Figures 7 and 8, the carrier arrangement 260 further includes a housing 268 that substantially covers the carrier 270 and the vessels 320 held thereby. However, access holes are provided through the housing 268 to provide access to certain stations S.

Turning now to Figures 7-9 and 11-18, actuation of the first probe arrangement 280 and the second probe arrangement 290 will be described in detail. In the example embodiment, the first probe arrangement 280 and the second probe arrangement 290 are actuated by linear actuators. In other embodiments, the actuation may be non-linear (e.g., rotational). As illustrated at Figures 7, 8, and 11-18, a displacement D 1 of the first probe arrangement 280 and a displacement D2 of the second probe arrangement 290 are defined. In the example embodiment, displacements D1 and D2 are vertical. In other embodiments, the displacements D1 and/or D2 may be non-vertical. A sign convention has been defined with respect to the displacements D1 and D2. In particular, a first direction D1+ and an opposite second direction D1- has been defined for displacement D1. Likewise, a first direction D2+ and a second direction D2- has been defined with respect to displacement D2. As depicted, directions D1+ and D2+ are upward, and directions D1- and D2- are downward.

The first probe arrangement 280 is actuated by a first actuator 282. Similarly the second probe arrangement 290 is actuated by a second actuator 292. The first actuator 282 includes a pulley 282P and a belt 282B. Likewise, the second actuator 292 includes a pulley 292P and a belt 292B. The first actuator 282 actuates a first probe platform 286 (e.g., a frame, a moveable frame, a mounting platform, etc.), and the second actuator 292 actuates a second probe platform 296 (e.g., a frame, a moveable frame, a mounting platform, etc.). In particular, the first probe platform 286 includes a platform attachment 286B that attaches to the belt 282B, and the second probe platform 296 includes a platform attachment 296B that attaches to the belt 292B. As illustrated at Figure 7, a first guide 284 (e.g., a first linear rail, a first linear bearing, etc.) is provided to guide the first probe arrangement 280 along displacement D1, and a second guide 294 (e.g., a second linear rail, a second linear bearing, etc.) is provided to guide the second probe arrangement 290 along the displacement D2. The first probe platform 286 includes a platform attachment 286A to attach to the moving portion of the first guide 284. Likewise, the second probe platform 296 includes a platform attachment 296A that attaches to the moving portion of the second guide 294. The actuators 282 and/or 292 may be powered by a motor that is connected to the computer 194 by a wiring harness 196. The actuators 282 and/or 292 and/or the motors that power them may be further connected to a power supply by the wiring harness 196.

As depicted, the displacement D1 allows movement of the first probe arrangement 280 along a single degree-of-freedom. As depicted, the displacement D1 allows movement of the first probe arrangement 280 along a single linear degree-of-freedom. As depicted, this degree-of-freedom is vertical. As depicted, this degree-of-freedom is also parallel to an axis of the carried probe(s). Similarly, as depicted, the displacement D2 allows movement of the second probe arrangement 290 along a single degree-of-freedom. As depicted, the displacement D2 allows movement of the second probe arrangement 290 along a single linear degree-of-freedom. As depicted, this degree-of-freedom is also vertical. As depicted, this degree-of-freedom is also parallel to an axis of the carried probe(s).

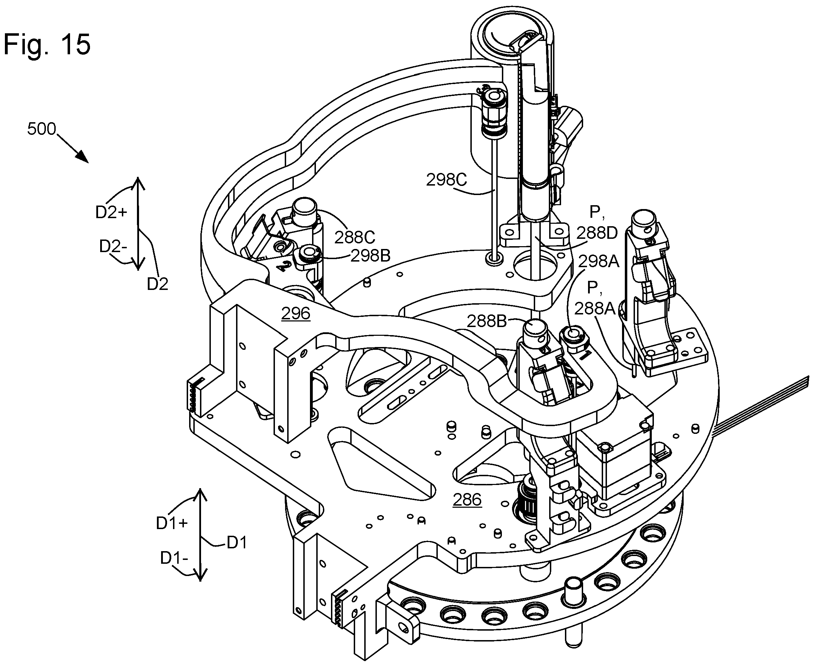

The first probe arrangement 280 may thereby be actuated to various positions along displacement D1. In particular, Figures 7, 8, 13, and 17 illustrate a first actuated position or range of positions DP1 of the first probe arrangement 280. Figures 11 and 15 illustrate a second actuated position or range of positions DP2 of the first probe arrangement 280. Figures 12, 14, 16, and 18 illustrate a third actuated position or range of positions DP3 of the first probe arrangement 280. The actuated position DP1 is a stowed position. The actuated position DP2 is used when positioning a wash station arrangement 400 at a washing position. Thus, in the depicted embodiment, the wash station arrangement 400 is positioned with the first probe arrangement 280 and washes probes P of the second probe arrangement 290. In other embodiments, the wash station arrangement 400 may be fixedly located with respect to the frame 262 of the carrier arrangement 260 and thereby be fixedly located with respect to the frame of the instrument 100 and thereby be located independent of the first probe arrangement 280. The actuated position DP3 is illustrated at Figures 12, 14, 16, and 18. The actuated position DP3 is a deployed position. In the depicted embodiment, the actuated position DP3 is a dispensing position. As illustrated at Figure 12, the spin-mixers 278, including a drive system with pulleys 278P, are rotationally mounted on the first probe platform 286. The actuated position DP3 is further a deployed position for the spin-mixers 278.

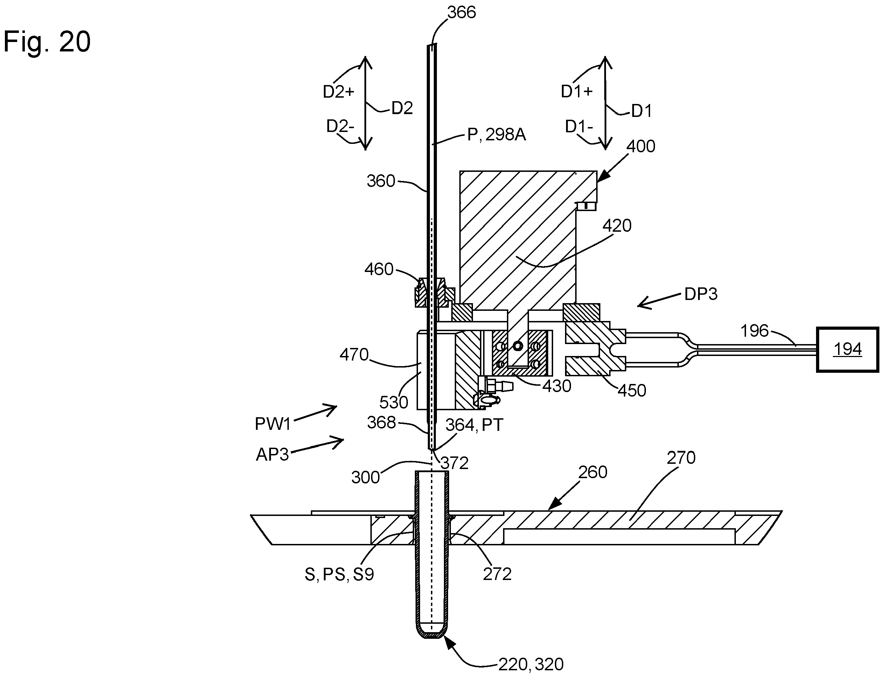

The second probe arrangement 290 may also be actuated to a plurality of positions. In particular, the second probe arrangement 290 may be actuated along displacement D2 to a first actuated position or range of positions AP1, a second actuated position (e.g., a washing position) or range of positions AP2, a third actuated position or range of positions AP3, and a fourth actuated position (e.g., an operating position) or range of positions AP4. As illustrated at Figures 7, 8, 13, and 17, the first actuated position AP1 is a stowed position. As illustrated at Figures 11 and 15, the second actuated position AP2 is a probe wash position. As illustrated at Figure 20, the third actuated position AP3 is an approach position or a retreat position where probe assemblies 298A, 298B, and/or 298C are approaching toward or retreating from the vessel 320. The fourth actuated position AP4 is illustrated at Figures 12, 14, 16, and 18. The fourth actuated position AP4 is an aspirating position.

As mentioned above, in certain embodiments, the actuated positions AP1, AP2, AP3, AP4, DP1, DP2, and DP3 may vary within a range of position. For example, when aspirating, a probe tip PT may follow a fluid level within the vessel 320 down as fluid is removed from the vessel 320. Thus, the aspirating position AP4 moves in the direction D2-as aspirating progresses.

As mentioned above, the first probe arrangement 280 includes probe assemblies 288A, 288B, 288C, and 288D. In the discussion below, probe assemblies 288A, 288B, 288C, and 288D may be generically referred to as probe assembly 288. Likewise, the second probe arrangement 290 includes probe assemblies 298A, 298B, and 298C. Probe assemblies 298A, 298B, and 298C may be generically referred to as probe assembly 298.

In describing the details of the wash station arrangement 400, the probe assembly 298 is described and illustrated. The wash station arrangement 400 may be adapted to the various other probes P, described and/or mentioned herein.

The probe assembly 298 is attached to the probe platform 296 of the probe arrangement 290 at a platform attachment 296P. In the depicted embodiment, the platform attachment 296P is spring-loaded and thereby provides protection to the probe assembly 298 during a collision. Such collisions are typically inadvertent. In other embodiments, the platform attachment 296P may fixedly attached the probe assembly 298 to the probe platform 296. As the probe assembly 298 is attached to the probe platform 296, the probe assembly 298 follows the probe platform 296 when the probe arrangement 290 is actuated. In the example embodiment, the probe platform 296 is guided along linear displacement D2. Thus, the probe assembly 298 also moves along displacements D2.

As illustrated at Figures 7, 8, 11-14, and 20, a probe path 300 is defined when the probe arrangement 290 moves along displacements D2. At Figures 7 and 8, the probe path 300 is shown as though a hidden line and therefor projects through various components that are in front of it. In normal operation of the depicted example analyzer 100, the probe path 300 includes a single degree-of-freedom. In other embodiments, the probe path 300 may be driven by multiple actuators and thereby include multiple degrees-of-freedom. The single degree-of-freedom of the depicted embodiment is sufficient to provide actuation to the probe assembly 298 for accessing the various probe receiving stations PS of the carrier arrangement 260 (see Figure 10). However, the carrier arrangement 260 does not include probe washing accommodation in the depicted embodiment. To accommodate the single degree-of-freedom of the probe assembly 298, the wash station arrangement 400 includes a degree-of-freedom to move a probe washer 470 of the wash station arrangement 400 into and out of the probe path 300 and thereby allow washing of the probe assembly 298 when the probe washer 470 of the wash station arrangement 400 is on the probe path 300 and further allow the probe assembly 298 to reach the probe receiving stations PS of the carrier arrangement 260.

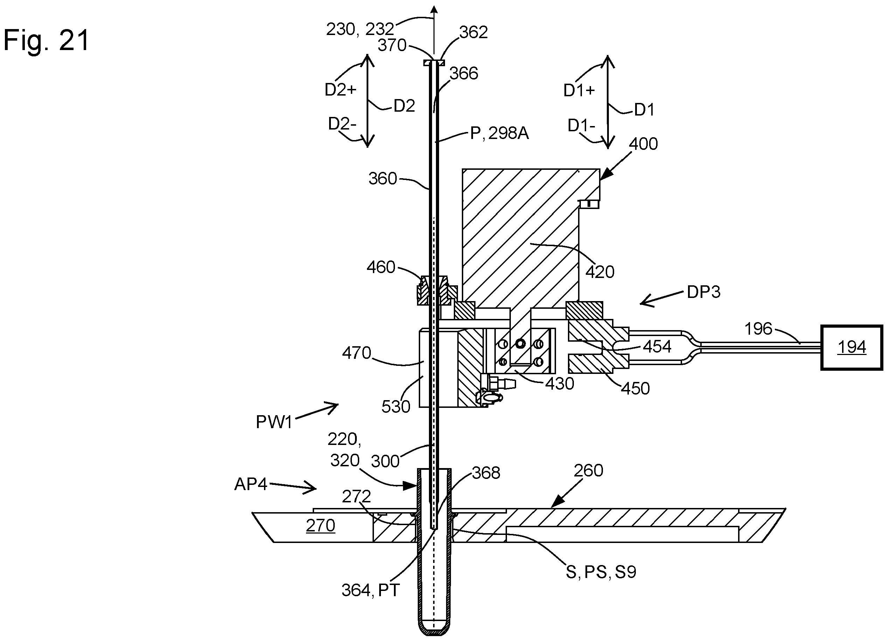

Turning now to Figure 20, the probe assembly 298 will be described in detail. The probe assembly 298 includes a probe body 360 that extends from a proximal end 362 to a distal end 364. The probe body 360 is tubular (i.e. hollow) in the depicted embodiment. The probe body 360 is substantially cylindrical in the depicted embodiment. The distal end 364 of the probe body 360 coincides with the probe tip PT. The probe body 360 includes an internal portion 366 and an external portion 368, and the probe P is thereby a hollow probe. The internal portion 366 provides a passage through the probe body 360 from the proximal end 362 to the distal end 364. An opening 370 (see Figure 21) at the proximal end 362 provides access to the internal portion 366, and an opening 372 (see Figure 20) at the distal end 364 provides access to the internal portion 366.

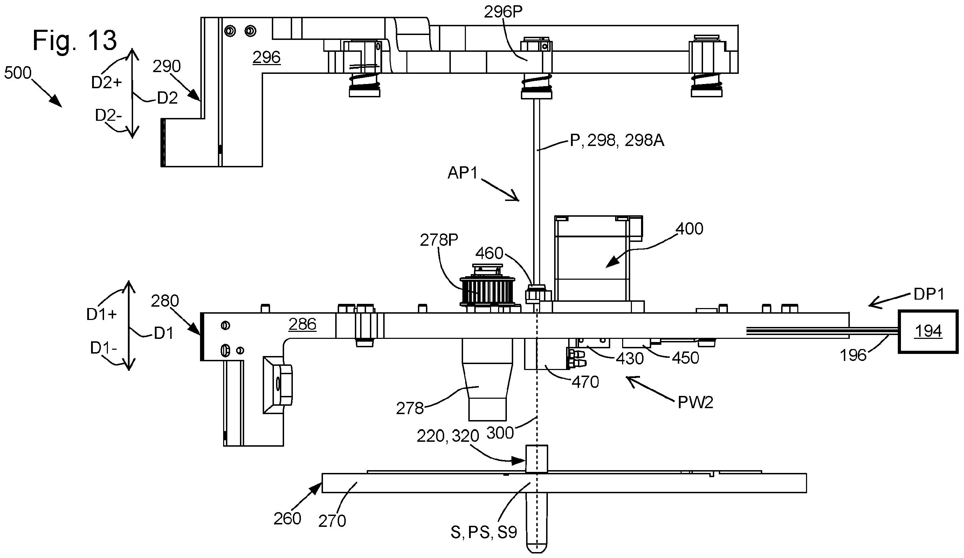

As depicted at Figures 13-21, the mount 422 also includes or has mounted to it a probe guide 460. As illustrated at Figures 19-21, the probe guide 460 may guide the probe P, 298 and thereby keep the probe P, 298 on the probe path 300.

Turning now to Figure 12, certain plumbing related to the probe assembly 298 will be described in detail. As depicted, the proximal end 262 of the probe body 360 is connected to various plumbing. For use as an aspirate probe assembly 298, the plumbing includes a pump 316 (e.g., a vacuum pump, a peristaltic pump) to aspirate fluid from the vessel 320. The aspirated fluid is thereby pumped to a waste fluid disposal 314. Valves, Ts, and various plumbing may be used to provide selective fluid communication between the probes P and various disposals 314 and supplies 304.

## Claims

1. An immunoassay diagnostic system (100) configured to perform a plurality of assay types (200, 600) and thereby detect analytes (244, 644) in patient samples (224, 624) by at least combining each of the patient samples (224, 624) with at least one reagent (216, 232, 616) and then washing away at least unreacted components (230, 630) of the patient sample, the immunoassay diagnostic system (100) comprising:
a reaction cell (220, 320) configured to hold the patient sample and the at least one reagent (216, 232, 616);
a washing arrangement (176);
a first set of probes (228, 288A, 288B, 288C, 628) and a second set of probes (218, 298A, 298B, 298C, 618); and
a controller (194) for controlling the immunoassay diagnostic system (100) configured to:
cause the washing arrangement (176) to wash away at least the unreacted components (230, 630) of the patient sample from the reaction cell (220, 320) by a multiple number of wash actions (206, 210, 606), cause the washing arrangement (176) to wash the reaction cell (220, 320) within a predetermined timed sequence (Tww), and to set the number of the wash actions (206, 210, 606) to correspond with the assay type; and
cause the first set of probes (228, 288A, 288B, 288C, 628) to dispense buffer solution (242, 642) into the reaction cell (220, 320) and the second set of probes (218, 298A, 298B, 298C, 618) to aspirate at least some of the buffer solution (242, 642) and at least some of the unreacted components (230, 630) of the patient sample from the reaction cell (220, 320), and thereby cause the first and second set of probes (228, 288A, 288B, 288C, 628; 218, 298A, 298B, 298C, 618) to perform a base number of the wash actions (206, 210, 606);
**characterised in that**, when performing a corresponding assay type, the controller (194) further causes a first probe (298B, 298C) of the second set of probes (218, 298A, 298B, 298C, 618) to dispense buffer solution (242, 642) into the reaction cell (220, 320) after initially aspirating the at least some of the buffer solution (242, 642) and the at least some of the unreacted components (230, 630) of the patient sample from the reaction cell (220, 320) and **in that** the controller (194) further causes the first probe of the second set of probes (218, 298A, 298B, 298C, 618) to subsequently aspirate at least some of the buffer solution (242, 642) and at least some of the unreacted components (230, 630) of the patient sample from the reaction cell (220, 320) after dispensing the buffer solution (242, 642) into the reaction cell (220, 320).

2. The immunoassay diagnostic system of claim 1, wherein the immunoassay diagnostic system (100) is configured to detect at least one antigen as one of the analytes (244, 644) in the patient samples (224, 624); and/or
wherein the immunoassay diagnostic system (100) is configured to detect at least one antibody as one of the analytes (244, 644) in the patient samples (224, 624); and/or
wherein the reaction cell (220, 320) includes a sample vessel (220, 320).

3. The immunoassay diagnostic system of claim 1 or 2, wherein when performing a corresponding assay type, the controller (194) further causes a second probe (298C, 298B) of the second set of probes (218, 298A, 298B, 298C, 618) to dispense buffer solution (242, 642) into the reaction cell (220, 320) after initially aspirating the at least some of the buffer solution (242, 642) and the at least some of the unreacted components (230, 630) of the patient sample from the reaction cell (220, 320) and wherein the controller (194) further causes the second probe of the second set of probes (218, 298A, 298B, 298C, 618) to subsequently aspirate at least some of the buffer solution (242, 642) and at least some of the unreacted components (230, 630) of the patient sample from the reaction cell (220, 320) after dispensing buffer solution (242, 642) into the reaction cell (220, 320).

4. The immunoassay diagnostic system of any of claims 1-3, wherein a first platform (286) is configured to move the first set of probes and a second platform (296) is configured to move the second set of probes (218, 298A, 298B, 298C, 618); and/or
wherein the first set of probes is configured for actuation about a first single linear degree-of-freedom (D1) and the second set of probes (218, 298A, 298B, 298C, 618) is configured for actuation about a second single linear degree-of-freedom (D2);
wherein, optionally, the first single linear degree-of-freedom and the second single linear degree-of-freedom are each vertical.

5. The immunoassay diagnostic system of any of claims 1-4, further comprising a plurality of the reaction cells, wherein the washing arrangement (176) includes a plurality of stations (S), wherein the washing arrangement (176) is configured to sequentially move each of the plurality of the reaction cells to each of the plurality of stations within the predetermined timed sequence (Tww),
wherein, optionally, the washing arrangement (176) includes a carrier (270) with a plurality of holders (272), wherein each of the holders is configured to hold a corresponding one of the plurality of the reaction cells, and wherein the carrier is configured to sequentially move each of the plurality of the holders to each of the plurality of stations within the predetermined timed sequence (Tww) and thereby sequentially move each of the plurality of the reaction cells to each of the plurality of stations within the predetermined timed sequence (Tww).

6. The immunoassay diagnostic system of claim 5, wherein the carrier is configured to move with a single degree-of-freedom;
wherein, optionally, the single degree-of-freedom is rotational about an axis (A1) of the washing arrangement (176);
wherein, optionally, the axis of the washing arrangement (176) is vertical.

7. The immunoassay diagnostic system of any of claims 1-6, wherein the washing arrangement (176) includes at least one magnetic collecting structure (226, 236) configured to restrain magnetic components (222) of the at least one reagent (216, 232, 616) and thereby retain within the reaction cell (220, 320) the at least one reagent (216, 232, 616) and portions of the patient sample attached to the at least one reagent (216, 232, 616) at least when the unreacted components (230, 630) of the patient sample are washed away by the wash actions (206, 210, 606).

8. The immunoassay diagnostic system of any of claims 1-3, wherein the washing arrangement (176) includes at least one magnetic collecting structure (226, 236) configured to restrain magnetic components (222) of the at least one reagent (216, 232, 616) and thereby retain within the reaction cell (220, 320) the at least one reagent (216, 232, 616) and portions of the patient sample attached to the at least one reagent (216, 232, 616) at least when the unreacted components (230, 630) of the patient sample are washed away by the wash actions (206, 210, 606) and wherein the at least one magnetic collecting structure is configured to restrain the magnetic components of the at least one reagent (216, 232, 616) when any of the second set of probes (218, 298A, 298B, 298C, 618) initially aspirates, dispenses, and subsequently aspirates.

9. The immunoassay diagnostic system of claim 8, wherein the at least one magnetic collecting structure is configured to continuously restrain the magnetic components of the at least one reagent (216, 232, 616) while the reaction cell (220, 320) is at a station (S17, S25) where any of the second set of probes (218, 298A, 298B, 298C, 618) initially aspirates, dispenses, and subsequently aspirates; or
wherein the at least one magnetic collecting structure is configured to continuously restrain the magnetic components of the at least one reagent (216, 232, 616) while any of the second set of probes (218, 298A, 298B, 298C, 618) initially aspirates, dispenses, and subsequently aspirates.

10. The immunoassay diagnostic system of any of claims 1-9, wherein the controller (194) is a computer (194) and the immunoassay diagnostic system (100) further comprises a non-transitory computer readable medium (198) having stored thereon data (188) operable to configure the computer (194) to:
read an assay protocol file (184) corresponding to the at least one reagent (216, 232, 616); and
transmit instructions to the washing arrangement (176) for the washing arrangement (176) to set the number of the wash actions (206, 210, 606).

11. The immunoassay diagnostic system of any of claims 1 to 3, wherein the controller (194) is a computer (194) and the immunoassay diagnostic system (100) further comprises a non-transitory computer readable medium (198) having stored thereon data (188) operable to configure the computer (194) to:
read an assay protocol file (184) corresponding to the at least one reagent (216, 232, 616); and
transmit instructions to the washing arrangement (176) for the washing arrangement (176) to set the number of the wash actions (206, 210, 606);
wherein the instructions include timed signals to one or more valves in fluid communication with the second set of probes (218, 298A, 298B, 298C, 618).

12. The immunoassay diagnostic system of any of claims 5-6, wherein the controller (194) is a computer (194) and the immunoassay diagnostic system (100) further comprises a non-transitory computer readable medium (198) having stored thereon data (188) operable to configure the computer (194) to:
individually identify each of the plurality of the reaction cells, the corresponding at least one reagent (216, 232, 616) contained therein, and a current corresponding station of the plurality of stations thereat; and
transmit instructions to the washing arrangement (176) for the washing arrangement (176) to set the number of the wash actions (206, 210, 606) at each of a plurality of wash stations (S17, S25) of the plurality of stations;
wherein the number of the wash actions (206, 210, 606) corresponds to each of the plurality of the reaction cells at each of the plurality of wash stations.

13. The immunoassay diagnostic system of claim 12, wherein the instructions include timed signals to one or more valves in fluid communication with the second set of probes (218, 298A, 298B, 298C, 618); and/or
wherein the data is further operable to configure the computer (194) to read an assay protocol file (184) corresponding to the at least one reagent (216, 232, 616) of each of the plurality of the reaction cells.

14. The immunoassay diagnostic system of any of claims 1-4, wherein the controller (194) is further configured to cause the second set of probes (218, 298A, 298B, 298C, 618) to descend into the reaction cell (220, 320) while aspirating, then raise within the reaction cell (220, 320), then suspend aspiration, then resume aspiration, then again descend into the reaction cell (220, 320) while aspirating, and then raise out of the reaction cell (220, 320) while aspirating.

15. The immunoassay diagnostic system of any of claims 1-3, wherein the controller (194) is further configured to cause the second set of probes (218, 298A, 298B, 298C, 618) to descend into the reaction cell (220, 320) while aspirating, then raise within the reaction cell (220, 320), then suspend aspiration, then dispense the buffer solution (242, 642) within the reaction cell (220, 320), then resume aspiration, then again descend into the reaction cell (220, 320) while aspirating, and then raise out of the reaction cell (220, 320) while aspirating.

## Patentansprüche

1. Immunoassay-Diagnosesystem (100), das derart konfiguriert ist, dass es eine Vielzahl von Testtypen (200, 600) durchführt und dadurch Analyten (244, 644) in Patientenproben (224, 624) nachweist, indem es mindestens jede der Patientenproben (224, 624) mit mindestens einem Reagenz (216, 232, 616) kombiniert und dann mindestens nicht umgesetzte Komponenten (230, 630) der Patientenprobe abwäscht, wobei das Immunoassay-Diagnosesystem (100) Folgendes umfasst:
eine Reaktionszelle (220, 320), die derart konfiguriert ist, dass sie die Patientenprobe und das mindestens eine Reagenz (216, 232, 616) hält;
eine Waschanordnung (176);
einen ersten Satz von Sonden (228, 288A, 288B, 288C, 628) und einen zweiten Satz von Sonden (218, 298A, 298B, 298C, 618); und
eine Steuerung (194) zum Steuern des Immunoassay-Diagnosesystems (100), die konfiguriert ist zum:
Veranlassen, dass die Waschanordnung (176) mindestens die nicht umgesetzten Komponenten (230, 630) der Patientenprobe aus der Reaktionszelle (220, 320) durch eine vielfache Anzahl von Waschvorgängen (206, 210, 606) abwäscht, Veranlassen, dass die Waschanordnung (176) die Reaktionszelle (220, 320) innerhalb einer vorbestimmten zeitlichen Abfolge (Tww) wäscht und die Anzahl der Waschvorgänge (206, 210, 606) so einstellt, dass sie dem Testtyp entsprechen; und
Veranlassen, dass der erste Satz von Sonden (228, 288A, 288B, 288C, 628) Pufferlösung (242, 642) in die Reaktionszelle (220, 320) abgibt und der zweite Satz von Sonden (218, 298A, 298B, 298C, 618) mindestens einen Teil der Pufferlösung (242, 642) und mindestens einen Teil der nicht umgesetzten Komponenten (230, 630) der Patientenprobe aus der Reaktionszelle (220, 320) aspiriert und dadurch den ersten und zweiten Satz von Sonden (228, 288A, 288B, 288C, 628; 218, 298A, 298B, 298C, 618) veranlasst, eine Basisanzahl der Waschvorgänge (206, 210, 606) durchzuführen;
**dadurch gekennzeichnet, dass** die Steuerung (194) beim Durchführen eines entsprechenden Testtyps ferner eine erste Sonde (298B, 298C) des zweiten Satzes von Sonden (218, 298A, 298B, 298C, 618) veranlasst, Pufferlösung (242, 642) in die Reaktionszelle (220, 320) abzugeben, nachdem zunächst mindestens ein Teil der Pufferlösung (242, 642) und mindestens ein Teil der nicht umgesetzten Komponenten (230, 630) der Patientenprobe aus der Reaktionszelle (220, 320) aspiriert werden, und dadurch, dass die Steuerung (194) ferner die erste Sonde des zweiten Satzes von Sonden (218, 298A, 298B, 298C, 618) veranlasst, anschließend mindestens einen Teil der Pufferlösung (242, 642) und mindestens einen Teil der nicht umgesetzten Komponenten (230, 630) der Patientenprobe aus der Reaktionszelle (220, 320) zu aspirieren, nachdem die Pufferlösung (242, 642) in die Reaktionszelle (220, 320) abgegeben wurde.

2. Immunoassay-Diagnosesystem nach Anspruch 1, wobei das Immunoassay-Diagnosesystem (100) derart konfiguriert ist, dass es mindestens ein Antigen als einen der Analyten (244, 644) in den Patientenproben (224, 624) nachweist; und/oder
wobei das Immunoassay-Diagnosesystem (100) derart konfiguriert ist, dass es mindestens einen Antikörper als einen der Analyten (244, 644) in den Patientenproben (224, 624) nachweist; und/oder
wobei die Reaktionszelle (220, 320) einen Probenbehälter (220, 320) beinhaltet.

3. Immunoassay-Diagnosesystem nach Anspruch 1 oder 2, wobei die Steuerung (194) beim Durchführen eines entsprechenden Testtyps ferner eine erste Sonde (298B, 298C) des zweiten Satzes von Sonden (218, 298A, 298B, 298C, 618) veranlasst, Pufferlösung (242, 642) in die Reaktionszelle (220, 320) abzugeben, nachdem zunächst mindestens ein Teil der Pufferlösung (242, 642) und mindestens ein Teil der nicht umgesetzten Komponenten (230, 630) der Patientenprobe aus der Reaktionszelle (220, 320) aspiriert werden und wobei die Steuerung (194) ferner die zweite Sonde des zweiten Satzes von Sonden (218, 298A, 298B, 298C, 618) veranlasst, anschließend mindestens einen Teil der Pufferlösung (242, 642) und mindestens einen Teil der nicht umgesetzten Komponenten (230, 630) der Patientenprobe aus der Reaktionszelle (220, 320) zu aspirieren, nachdem Pufferlösung (242, 642) in die Reaktionszelle (220, 320) abgegeben wurde.

4. Immunoassay-Diagnosesystem nach einem der Ansprüche 1-3, wobei eine erste Plattform (286) derart konfiguriert ist, dass sie den ersten Satz von Sonden bewegt und eine zweite Plattform (296) derart konfiguriert ist, dass sie den zweiten Satz von Sonden (218, 298A, 298B, 298C, 618) bewegt; und/oder
wobei der erste Satz von Sonden derart konfiguriert ist, dass er um einen ersten einzelnen linearen Freiheitsgrad (D1) betätigt wird, und der zweite Satz von Sonden (218, 298A, 298B, 298C, 618) derart konfiguriert ist, dass er um einen zweiten einzelnen linearen Freiheitsgrad (D2) betätigt wird;
wobei optional der erste einzelne lineare Freiheitsgrad und der zweite einzelne lineare Freiheitsgrad jeweils vertikal ausgerichtet sind.

5. Immunoassay-Diagnosesystem nach einem der Ansprüche 1-4, ferner umfassend eine Vielzahl von den Reaktionszellen, wobei die Waschanordnung (176) eine Vielzahl von Stationen (S) beinhaltet, wobei die Waschanordnung (176) derart konfiguriert ist, dass sie jede der Vielzahl von den Reaktionszellen innerhalb der vorbestimmten zeitlichen Abfolge (Tww) sequentiell zu jeder der Vielzahl von Stationen bewegt;
wobei optional die Waschanordnung (176) einen Träger (270) mit einer Vielzahl von Haltern (272) beinhaltet, wobei jeder der Halter derart konfiguriert ist, dass er eine entsprechende Reaktionszelle aus einer der Vielzahl von den Reaktionszellen hält, und wobei der Träger derart konfiguriert ist, dass er jeden der Vielzahl von Haltern sequentiell zu jeder der Vielzahl von Stationen innerhalb der vorbestimmten zeitlichen Abfolge (Tww) bewegt und dadurch jede der Vielzahl von den Reaktionszellen sequentiell zu jeder der Vielzahl von Stationen innerhalb der vorbestimmten zeitlichen Abfolge (Tww) bewegt.

6. Immunoassay-Diagnosesystem nach Anspruch 5, wobei der Träger derart konfiguriert ist, dass er sich mit einem einzelnen Freiheitsgrad bewegt;
wobei optional der einzelne Freiheitsgrad um eine Achse (A1) der Waschanordnung (176) drehbar ist;
wobei optional die Achse der Waschanordnung (176) vertikal ausgerichtet ist.

7. Immunoassay-Diagnosesystem nach einem der Ansprüche 1-6, wobei die Waschanordnung (176) mindestens eine magnetische Sammelstruktur (226, 236) beinhaltet, die derart konfiguriert ist, dass sie magnetische Komponenten (222) des mindestens einen Reagenz (216, 232, 616) zurückhält und dadurch innerhalb der Reaktionszelle (220, 320) das mindestens eine Reagenz (216, 232, 616) und Abschnitte der Patientenprobe, die an das mindestens eine Reagenz (216, 232, 616) gebunden sind, mindestens dann zurückzuhalten, wenn die nicht umgesetzten Komponenten (230, 630) der Patientenprobe durch die Waschvorgänge (206, 210, 606) abgewaschen werden.

8. Immunoassay-Diagnosesystem nach einem der Ansprüche 1-3, wobei die Waschanordnung (176) mindestens eine magnetische Sammelstruktur (226, 236) beinhaltet, die derart konfiguriert ist, dass sie magnetische Komponenten (222) des mindestens einen Reagenz (216, 232, 616) zurückhält und dadurch innerhalb der Reaktionszelle (220, 320) das mindestens eine Reagenz (216, 232, 616) und Abschnitte der Patientenprobe, die an das mindestens eine Reagenz (216, 232, 616) gebunden sind, mindestens dann zurückzuhalten, wenn die nicht umgesetzten Komponenten (230, 630) der Patientenprobe durch die Waschvorgänge (206, 210, 606) abgewaschen werden und wobei die mindestens eine magnetische Sammelstruktur derart konfiguriert ist, dass sie die magnetischen Komponenten des mindestens einen Reagenz (216, 232, 616) zurückhält, wenn ein beliebiger des zweiten Satzes von Sonden (218, 298A, 298B, 298C, 618) zunächst aspiriert, abgibt und anschließend aspiriert.

9. Immunoassay-Diagnosesystem nach Anspruch 8, wobei die mindestens eine magnetische Sammelstruktur derart konfiguriert ist, dass sie die magnetischen Komponenten des mindestens einen Reagenz (216, 232, 616) kontinuierlich zurückhält, während sich die Reaktionszelle (220, 320) an einer Station (S17, S25) befindet, an der ein beliebiger des zweiten Satzes von Sonden (218, 298A, 298B, 298C, 618) zunächst aspiriert, abgibt und anschließend aspiriert; oder
wobei die mindestens eine magnetische Sammelstruktur derart konfiguriert ist, dass sie die magnetischen Komponenten des mindestens einen Reagenz (216, 232, 616) kontinuierlich zurückhält, während ein beliebiger des zweiten Satzes von Sonden (218, 298A, 298B, 298C, 618) zunächst aspiriert, abgibt und anschließend aspiriert.

10. Immunoassay-Diagnosesystem nach einem der Ansprüche 1-9, wobei die Steuerung (194) ein Computer (194) ist und das Immunoassay-Diagnosesystem (100) ferner ein nicht-flüchtiges computerlesbares Medium (198) umfasst, auf dem Daten (188) gespeichert sind, die so verarbeitet werden können, dass der Computer (194) konfiguriert ist zum:
Lesen einer Testprotokolldatei (184), die dem mindestens einen Reagenz (216, 232, 616) entspricht; und
Übertragen von Anweisungen an die Waschanordnung (176), damit die Waschanordnung (176) die Anzahl der Waschvorgänge (206, 210, 606) einstellt.

11. Immunoassay-Diagnosesystem nach einem der Ansprüche 1-3, wobei die Steuerung (194) ein Computer (194) ist und das Immunoassay-Diagnosesystem (100) ferner ein nicht-flüchtiges computerlesbares Medium (198) umfasst, auf dem Daten (188) gespeichert sind, die so verarbeitet werden können, dass der Computer (194) konfiguriert ist zum:
Lesen einer Testprotokolldatei (184), die dem mindestens einen Reagenz (216, 232, 616) entspricht; und
Übertragen von Anweisungen an die Waschanordnung (176), damit die Waschanordnung (176) die Anzahl der Waschvorgänge (206, 210, 606) einstellt;
wobei die Anweisungen zeitgesteuerte Signale an ein oder mehrere Ventile in Fluidverbindung mit dem zweiten Satz von Sonden (218, 298A, 298B, 298C, 618) beinhalten.

12. Immunoassay-Diagnosesystem nach einem der Ansprüche 5-6, wobei die Steuerung (194) ein Computer (194) ist und das Immunoassay-Diagnosesystem (100) ferner ein nicht-flüchtiges computerlesbares Medium (198) umfasst, auf dem Daten (188) gespeichert sind, die so verarbeitet werden können, dass der Computer (194) konfiguriert ist zum:
individuellen Identifizieren jeder der Vielzahl von den Reaktionszellen, wobei das entsprechende mindestens eine Reagenz (216, 232, 616) darin enthalten ist, und einer aktuellen entsprechenden Station der Vielzahl von Stationen darin; und
Übertragen von Anweisungen an die Waschanordnung (176), damit die Waschanordnung (176) die Anzahl der Waschvorgänge (206, 210, 606) an jeder einer Vielzahl von Waschstationen (S17, S25) der Vielzahl von Stationen einstellt;
wobei die Anzahl der Waschvorgänge (206, 210, 606) jeder der Vielzahl von den Reaktionszellen an jeder der Vielzahl von Waschstationen entspricht.

13. Immunoassay-Diagnosesystem nach Anspruch 12, wobei die Anweisungen zeitgesteuerte Signale an ein oder mehrere Ventile in Fluidverbindung mit dem zweiten Satz von Sonden (218, 298A, 298B, 298C, 618) beinhalten; und/oder
wobei die Daten ferner so verarbeitet werden können, dass sie den Computer (194) so konfigurieren, dass er eine Testprotokolldatei (184) liest, die dem mindestens einen Reagenz (216, 232, 616) jeder der Vielzahl von den Reaktionszellen entspricht.

14. Immunoassay-Diagnosesystem nach einem der Ansprüche 1-4, wobei die Steuerung (194) ferner derart konfiguriert ist, dass sie den zweiten Satz von Sonden (218, 298A, 298B, 298C, 618) veranlasst, während des Aspirierens in die Reaktionszelle (220, 320) abzusinken, dann innerhalb der Reaktionszelle (220, 320) anzusteigen, dann das Aspirieren zu unterbrechen, dann das Aspirieren wieder aufzunehmen, dann während des Aspirierens wieder in die Reaktionszelle (220, 320) abzusinken und dann während des Aspirierens aus der Reaktionszelle (220, 320) auszusteigen.

15. Immunoassay-Diagnosesystem nach einem der Ansprüche 1-3, wobei die Steuerung (194) ferner derart konfiguriert ist, dass sie den zweiten Satz von Sonden (218, 298A, 298B, 298C, 618) veranlasst, während des Aspirierens in die Reaktionszelle (220, 320) abzusinken, dann innerhalb der Reaktionszelle (220, 320) anzusteigen, dann das Aspirieren zu unterbrechen, dann die Pufferlösung (242, 642) innerhalb der Reaktionszelle (220, 320) abzugeben, dann das Aspirieren wieder aufzunehmen, dann während des Aspirierens wieder in die Reaktionszelle (220, 320) abzusinken und dann während des Aspirierens aus der Reaktionszelle (220, 320) auszusteigen.

## Revendications

1. Système de diagnostic par dosage immunologique (100) configuré pour réaliser une pluralité de types de dosages (200, 600) et pour détecter ainsi des analytes (244, 644) dans des échantillons de patient (224, 624) en combinant au moins chacun des échantillons de patient (224, 624) à au moins un réactif (216, 232, 616) puis en éliminant par lavage au moins des composants n'ayant pas réagi (230, 630) de l'échantillon de patient, le système de diagnostic par dosage immunologique (100) comprenant :
une cellule de réaction (220, 320) configurée pour maintenir l'échantillon de patient et l'au moins un réactif (216, 232, 616) ;
un ensemble de lavage (176) ;
un premier ensemble d'échantillons (228, 288A, 288B, 288C, 628) et un deuxième ensemble d'échantillons (218, 298A, 298B, 298C, 618) ; et
un dispositif de commande (194) destiné à commander le système de diagnostic par dosage immunologique (100) configuré pour :
amener l'ensemble de lavage (176) à éliminer par lavage au moins les composants n'ayant pas réagi (230, 630) de l'échantillon de patient hors de la cellule de réaction (220, 320) par un nombre multiple d'actions de levage (206, 210, 606), amener l'ensemble de lavage (176) à laver la cellule de réaction (220, 320) dans une séquence temporisée prédéterminée (Tww), et définir le nombre des actions de lavage (206, 210, 606) de manière à correspondre au type de dosage ; et
amener le premier ensemble d'échantillons (228, 288A, 288B, 288C, 628) à distribuer une solution tampon (242, 642) dans la cellule de réaction (220, 320) et le deuxième ensemble d'échantillons (218, 298A, 298B, 298C, 618) à aspirer au moins une certaine quantité de la solution tampon (242, 642) et au moins une certaine quantité des composants n'ayant pas réagi (230, 630) de l'échantillon de patient hors de la cellule de réaction (220, 320), et amener ainsi le premier et le deuxième ensemble d'échantillons (228, 288A, 288B, 288C, 628 ; 218, 298A, 298B, 298C, 618) à réaliser un nombre de base des actions de lavage (206, 210, 606) ;
**caractérisé en ce que**, lors de la réalisation d'un type de dosage correspondant, le dispositif de commande (194) amène en outre un premier échantillon (298B, 298C) du deuxième ensemble d'échantillons (218, 298A, 298B, 298C, 618) à distribuer de la solution tampon (242, 642) dans la cellule de réaction (220, 320) après l'aspiration initiale de l'au moins une certaine quantité de la solution tampon (242, 642) et de l'au moins une certaine quantité des composants n'ayant pas réagi (230, 630) de l'échantillon de patient hors de la cellule de réaction (220, 320), et **en ce que** le dispositif de commande (194) amène en outre le premier échantillon du deuxième ensemble d'échantillons (218, 298A, 298B, 298C, 618) à aspirer ultérieurement au moins une certaine quantité de la solution tampon (242, 642) et au moins une certaine quantité des composants n'ayant pas réagi (230, 630) de l'échantillon de patient hors de la cellule de réaction (220, 320) après la distribution de la solution tampon (242, 642) dans la cellule de réaction (220, 320).

2. Système de diagnostic par dosage immunologique selon la revendication 1, dans lequel le système de diagnostic par dosage immunologique (100) est configuré pour détecter au moins un antigène en tant qu'un des analytes (244, 644) dans les échantillons de patient (224, 624) ; et/ou
dans lequel le système de diagnostic par dosage immunologique (100) est configuré pour détecter au moins un anticorps en tant qu'un des analytes (244, 644) dans les échantillons de patient (224, 624) ; et/ou
dans lequel la cellule de réaction (220, 320) comporte un récipient pour échantillon (220, 320).

3. Système de diagnostic par dosage immunologique selon la revendication 1 ou 2, dans lequel, lors de la réalisation d'un type de dosage correspondant, le dispositif de commande (194) amène en outre un deuxième échantillon (298C, 298B) du deuxième ensemble d'échantillons (218, 298A, 298B, 298C, 618) à distribuer de la solution tampon (242, 642) dans la cellule de réaction (220, 320) après l'aspiration initiale de l'au moins une certaine quantité de la solution tampon (242, 642) et de l'au moins une certaine quantité des composants n'ayant pas réagi (230, 630) de l'échantillon de patient hors de la cellule de réaction (220, 320) et dans lequel le dispositif de commande (194) amène en outre le deuxième échantillon du deuxième ensemble d'échantillons (218, 298A, 298B, 298C, 618) à aspirer ultérieurement au moins une certaine quantité de la solution tampon (242, 642) et au moins une certaine quantité des composants n'ayant pas réagi (230, 630) de l'échantillon de patient hors de la cellule de réaction (220, 320) après la distribution de la solution tampon (242, 642) dans la cellule de réaction (220, 320).

4. Système de diagnostic par dosage immunologique selon l'une quelconque des revendications 1-3, dans lequel une première plateforme (286) est configurée pour déplacer le premier ensemble d'échantillons et une deuxième plateforme (296) est configurée pour déplacer le deuxième ensemble d'échantillons (218, 298A, 298B, 298C, 618) ;
et/ou
dans lequel le premier ensemble d'échantillons est configuré pour l'actionnement autour d'un premier degré de liberté (D1) linéaire unique et le deuxième ensemble d'échantillons (218, 298A, 298B, 298C, 618) est configuré pour l'actionnement autour d'un deuxième degré de liberté de mouvement linéaire unique (D2) ;
dans lequel en option le premier degré de liberté linéaire unique et le deuxième degré de liberté linéaire unique sont respectivement verticaux.

5. Système de diagnostic par dosage immunologique selon l'une quelconque des revendications 1 -4, comprenant en outre une pluralité des cellules de réaction, dans lequel l'ensemble de lavage (176) comporte une pluralité de stations (S), dans lequel l'ensemble de lavage (176) est configuré pour déplacer de manière séquentielle chacune de la pluralité des cellules de réaction à chacune de la pluralité de stations dans la séquence temporisée prédéterminée (Tww) ;
dans lequel, en option, l'ensemble de lavage (176) comporte un support (270) avec une pluralité de systèmes de maintien (272), dans lequel chacun des systèmes de maintien est configuré pour maintenir une cellule de réaction correspondante de la pluralité des cellules de réaction, et dans lequel le support est configuré pour déplacer de manière séquentielle chacun de la pluralité de systèmes de maintien à chacune de la pluralité de stations dans la séquence temporisée prédéterminée (Tww) et pour déplacer ainsi de manière séquentielle chacune de la pluralité des cellules de réaction à chacune de la pluralité de stations dans la séquence temporisée prédéterminée (Tww).

6. Système de diagnostic par dosage immunologique selon la revendication 5, dans lequel le support est configuré pour se déplacer avec un seul degré de liberté ;
dans lequel, en option, le seul degré de liberté est en rotation autour d'un axe (A1) de l'ensemble de lavage (176) ;
dans lequel, en option, l'axe de l'ensemble de lavage (176) est vertical.

7. Système de diagnostic par dosage immunologique selon l'une quelconque des revendications 1-6, dans lequel l'ensemble de lavage (176) comporte au moins une structure de collecte magnétique (226, 236) configurée pour limiter des composants magnétiques (222) de l'au moins un réactif (216, 232, 616) et pour ainsi retenir dans la cellule de réaction (220, 320) l'au moins un réactif (216, 232, 616) et des parties de l'échantillon de patient fixées à l'au moins un réactif (216, 232, 616) au moins lorsque les composants n'ayant pas réagi (230, 630) de l'échantillon de patient sont éliminés par lavage par les actions de lavage (206, 210, 606).

8. Système de diagnostic par dosage immunologique selon l'une quelconque des revendications 1-3, dans lequel l'ensemble de lavage (176) comporte au moins une structure de collecte magnétique (226, 236) configurée pour limiter des composants magnétiques (222) de l'au moins un réactif (216, 232, 616) et pour ainsi retenir dans la cellule de réaction (220, 320) l'au moins un réactif (216, 232, 616) et des parties de l'échantillon de patient fixés à l'au moins un réactif (216, 232, 616) au moins lorsque les composants n'ayant pas réagi (230, 630) de l'échantillon de patient sont éliminés par lavage par les actions de lavage (206, 210, 606) et
dans lequel l'au moins une structure de collecte magnétique est configurée pour limiter les composants magnétiques de l'au moins un réactif (216, 232, 616) lorsqu'un quelconque échantillon du deuxième ensemble d'échantillons (218, 298A, 298B, 298C, 618) aspire initialement, distribue et aspire ultérieurement.

9. Système de diagnostic par dosage immunologique selon la revendication 9, dans lequel l'au moins une structure de collecte magnétique est configurée pour limiter en continu les composants magnétiques de l'au moins un réactif (216, 232, 616) tandis que la cellule de réaction (220, 320) est sur une station (S17, S25) où un quelconque échantillon du deuxième ensemble d'échantillons (218, 298A, 298B, 298C, 618) aspire initialement, distribue et aspire ultérieurement ; ou
dans lequel l'au moins une structure de collecte magnétique est configurée pour limiter en continu les composants magnétiques de l'au moins un réactif (216, 232, 616) tandis qu'un échantillon quelconque du deuxième ensemble d'échantillons (218, 298A, 298B, 298C, 618) aspire initialement, distribue et aspire ultérieurement.

10. Système de diagnostic par dosage immunologique selon l'une quelconque des revendications 1-9, dans lequel le dispositif de commande (194) est un ordinateur (194) et le système de diagnostic par dosage immunologique (100) comprend en outre un support lisible par ordinateur non transitoire (198) stockant des données (188), pouvant fonctionner pour configurer l'ordinateur (194) pour :
lire un fichier de protocole de dosage (184) correspondant à l'au moins un réactif (216, 232, 616) ; et
transmettre des instructions à l'ensemble de lavage (176) pour l'ensemble de lavage (176) pour définir le nombre des actions de lavage (206, 210, 606).

11. Système de diagnostic par dosage immunologique selon l'une quelconque des revendications 1 à 3, dans lequel le dispositif de commande (194) est un ordinateur (194) et le système de diagnostic par dosage immunologique (100) comprend en outre un support lisible par ordinateur (non transitoire (198) comportant des données stockées (188) pouvant fonctionner pour configurer l'ordinateur (194) pour :
lire un fichier de protocole de dosage (184) correspondant à l'au moins un réactif (216, 232, 616) ; et
transmettre des instructions à l'ensemble de lavage (176) pour l'ensemble de lavage (176) pour définir le nombre des actions de lavage (206, 210, 606) ;
dans lequel les instructions comportent des signaux temporisés à une ou plusieurs soupapes en communication fluidique avec le deuxième ensemble d'échantillons (218, 298A, 298B, 298C, 618).

12. Système de diagnostic par dosage immunologique selon les revendications 5-6, dans lequel le dispositif de commande (194) est un ordinateur (194) et le système de diagnostic par dosage immunologique (100) comprend en outre un support lisible par ordinateur non transitoire (198) présentant des données stockées (188) pouvant fonctionner pour configurer l'ordinateur (194) pour :
identifier individuellement chacune de la pluralité des cellules de réaction, l'au moins un réactif (216, 232, 616) correspondant contenu dans celles-ci, et une station correspondante actuelle de la pluralité de stations sur celle-ci ; et
transmettre des instructions à l'ensemble de lavage (176) pour l'ensemble de lavage (176) pour définir le nombre des actions de lavage (206, 210, 606) sur chacune de la pluralité de stations de lavage (S17, S25) de la pluralité de stations ;
dans lequel le nombre des actions de lavage (206, 210, 606) correspond à chacune de la pluralité des cellules de réaction sur chacune de la pluralité de stations de lavage.

13. Système de diagnostic par dosage immunologique selon la revendication 12, dans lequel les instructions comportent des signaux temporisés à une ou plusieurs soupapes en communication fluidique avec le deuxième ensemble d'échantillons (218, 298A, 298B, 298C, 618) ; et/ou
dans lequel les données peuvent en outre fonctionner pour configurer l'ordinateur (194) pour lire un fichier de protocole de dosage (184) correspondant à l'au moins une réaction (216, 232, 616) de chacune de la pluralité de cellules de réaction.

14. Système de diagnostic par dosage immunologique selon l'une quelconque des revendications 1 - 4, dans lequel le dispositif de commande (194) est configuré en outre pour amener le deuxième ensemble d'échantillons (218, 298A, 298B, 298C) à descendre dans la cellule de réaction (220, 320) lors de l'aspiration, puis à remonter dans la cellule de réaction (220, 230), puis à suspendre l'aspiration, puis à reprendre l'aspiration, puis à descendre à nouveau dans la cellule de réaction (220, 320) lors de l'aspiration, puis à remonter hors de la cellule de réaction (220, 320) lors de l'aspiration.

15. Système de diagnostic par dosage immunologique selon l'une quelconque des revendications 1 - 3, dans lequel le dispositif de commande (194) est configuré en outre pour amener le deuxième ensemble d'échantillons (218, 298A, 298B, 298C, 618) à descendre dans la cellule de réaction (220, 230) lors de l'aspiration, puis à remonter dans la cellule de réaction (220, 320), puis à suspendre l'aspiration, puis à distribuer la solution tampon (242, 642) dans la cellule de réaction (220, 320), puis à reprendre l'aspiration, puis à descendre à nouveau dans la cellule de réaction (220, 320) lors de l'aspiration, puis à remonter hors de la cellule de réaction (220, 320) lors de l'aspiration.
